# EUROPEAN PATENT SPECIFICATION

(11) **EP 0 387 923 B1**
(45) Date of publication and mention of the grant of the patent: **16.02.1994**
(21) Application number: 90109068.8
(22) Date of filing: 31.03.1987
(51) Int. Cl.: C07D 213/64, C07D 239/00, A01N 43/00

(54) **Fungicides**
Fungizide
Fongicides

(30) Priority: 17.04.1986 GB 8609454; 23.12.1986 GB 8630825
(43) Date of publication of application: 19.09.1990
(62) Divisional of application: 87302795.7
(73) Proprietor: ZENECA LIMITED, London W1Y 6LN (GB)
(72) Inventor: Anthony, Vivienne Margaret, Maidenhead, Berks. (GB); Clough, John Martin, Marlow, Bucks. (GB); DeFraine, Paul, Wokingham, Berks. (GB); Godfrey, Christopher Richard Ayles, Bracknell, Berks. (GB); Ferguson, Ian, Walsden, Todmorden, Lancashire (GB); Crowley, Patrick Jelf, Crowthorne, Berks. (GB); Hutchings, Michael Gordon, Holcombe, Bury BL8 4PD (GB)
(74) Representative: Houghton, Malcolm John

(56) References cited:
- EP-A- 0 178 826

## Description

The present invention relates to benzoyl formate derivatives which may be used in the preparation of acrylic acid derivatives useful in agriculture (especially as fungicides but also as plant growth regulators, insecticides and nematocides) and to processes for preparing them.

EP-A-0178826 describes fungicidal alkyl 2-(substituted)phenyl-3-alkoxyacrylates and lists the compound (E)-methyl 2-[2-(5-trifluoromethylpyridin-2-yloxy)phenyl]-3-methoxyacrylate and certain phenyl substituted analogues. It refers also to corresponding keto esters as intermediates.

European Patent Application No. 87302795.7 describes acrylic acid derivatives having the formula (I): 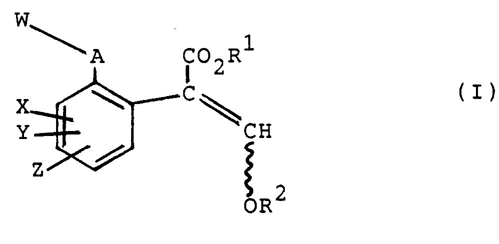 and stereoisomers thereof, wherein W is a substituted pyridinyl or substituted pyrimidinyl group linked to A by any of its ring carbon atoms; A is either an oxygen atom or S(O)ₙ wherein n is 0, 1 or 2; X, Y and Z, which are the or different, are hydrogen or halogen atoms, or hydroxy, optionally substituted alkyl (including haloalkyl), optionally substituted alkenyl, optionally substituted alkynyl, optionally substituted alkoxy, (including haloalkoxy), optionally substituted alkylthio, optionally substituted aryloxy, optionally substituted arylal- koxy, optionally substituted acyloxy, optionally substituted amino, optionally substituted acylamino, nitro, cyano, -C0₂ R³, -CONR4R5, -COR⁶ or -S(O)ₘR⁷ (wherein m is 0, 1 or 2) groups, or any two of the groups X, Y and Z, when they are in adjacent positions on the phenyl ring, may join to form a fused ring, either aromatic or aliphatic, optionally containing one or more heteroatoms; R¹ and R², which are the same or different, are optionally substituted alkyl (including fluoroalkyl) groups provided that when W is 5-trifluoromethylpyridin-2-yl, A is oxygen, X is hydrogen, and R¹ and R² are both methyl, Y and Z are not both hydrogen, Y is not F, Cl, methyl, nitro, 5-CF₃, 5-SCH₃ or 4-(CH₃)₂N if Z is hydrogen and Y and Z together are not 3-nitro-5-chloro, 3,5-dinitro, 4,5-dimethoxy or 4,5-methylenedioxy; and R³, R⁴, R⁵, R⁶ and R⁷, which are the same or different, are hydrogen atoms or optionally substituted alkyl, optionally substituted cycloalkyl, optionally substituted cycloalkylalkyl, optionally substituted alkenyl, optionally substituted alkynyl, optionally substituted aryl or optionally substituted aralkyl groups; and metal complexes thereof.

The above compounds contain at least one carbon-carbon double bond, and are sometimes obtained in the form of mixtures of geometric isomers. However, these mixtures can be separated into individual isomers.

The invention provides a compound having the formula (XV): 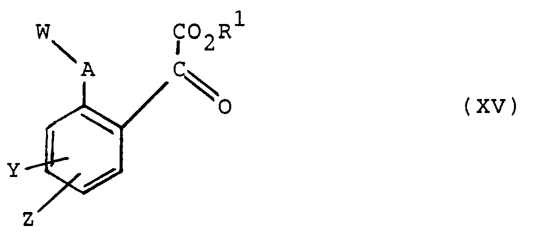 wherein W is a substituted pyridinyl or substituted pyrimidinyl group linked to A by any of its ring carbon atoms and bearing one or more substituents selected from halogen atoms, C₁ -₆ alkyl itself optionally substituted with halo, phenyl or phenoxy, C₂-₆ alkenyl, phenyl(C₂-₆)alkenyl, C₂-₆ alkynyl, C₁-₆ alkoxy itself optionally substituted with halo, phenyl or phenoxy, phenoxy, pyridinyloxy, pyrimidinyloxy, phenyl, pyridinyl, pyrimidinyl, nitro, cyano, -NR'R" -NHCOR', -CONR'R", -OCOR', -C0₂R', -COR' or S(O)ₘR' groups, wherein m is 0, 1 or 2 and R' and R" are as defined below; A is either an oxygen atom or S(O)ₙ wherein n is 0, 1 or 2; Y and Z, which are the same or different, are hydrogen or halogen atoms, or C₁₋₆ alkyl, C₁₋₄ alkyl optionally substituted with halo, C₁₋₆ alkoxy or phenyl, C₂-₆ alkenyl, phenyl, C₂-₆ alkynyl, C₁₋₆ alkoxy, C₁₋₄ alkoxy optionally substituted with halo or C₁₋₆ alkoxy, phenoxy, phenyl(C₁₋₆)alkoxy, -OCOR', -NR'R", -NHCOR', nitro, cyano, -C0₂R³, -CONR⁴R⁵, or -COR⁶ groups, wherein R', R" and R³ to R⁶ are as defined below; R¹ is a C, -4 alkyl or C, -4 haloalkyl (including fluoroalkyl) group; R³, R⁴, R⁵ and R⁶, which are the same or different, are hydrogen atoms or C₁₋₆ alkyl, C₃₋₆ cycloalkyl, C₃₋₆ cycloalkyl-(C₁₋₄)alkyl, C₂-₆ alkenyl, C₂-₆ alkynyl, phenyl or phenyl(C₁₋₆)alkyl; R' and R" are independently hydrogen, C₁₋₄ alkyl, C₁₋₄ alkoxy, C₁₋₄ alkylthio, C₃₋₆ cycloalkyl, C₃₋₆ cycloalkyl(C₁₋₄)alkyl, phenyl or benzyl, in which the phenyl and benzyl groups are optionally substituted with halogen, C₁ -₄ alkyl or C₁₋₄ alkoxy; and wherein any of the foregoing phenyl or heteroaryl moieties of Y and Z and of the substituents of W, except where otherwise stated for R' and R", are optionally substituted by one or more of the following: halogen, hydroxy, C₁₋₄ alkyl, C₁₋₄ alkoxy, halo(C₁₋₄)alkyl, halo(C₁₋₄)alkoxy, C₁₋₄ alkylthio, C₁₋₄ alkoxy-(C₁₋₄)alkyl, C₃₋₆ cycloalkyl, C₃₋₆ cycloalkyl(C1-4)alkyl, phenyl, phenoxy, phenyl(C₁₋₄)alkyl, phenyl(C₁-₄)-alkoxy, phenoxy(C₁-₄)alkyl, cyano, thiocyanato, nitro, -NR'R", -NHCOR', -NHCONR'R", -CONR'R", -COOR', -OS0₂ R', -S0₂ R', -COR', -OCOR', -CR' = NR" or -N = CR'R" wherein R' and R" are as defined above; provided that when W is 5-trifluoromethylpyridin-2-yl, A is oxygen and R¹ is methyl, Y and Z are not both hydrogen, Y is not F, Cl, methyl, nitro, 5-CF₃, or 4-(CH₃)₂N if Z is hydrogen and Y and Z together are not 3-nitro-5-chloro, 3,5-dinitro or 4,5-dimethoxy.

Alkyl groups, wherever present as a group or moiety in, for example, "alkoxy", "alkylthio" and "aralkyl", can be in the form of straight or branched chains, and contain 1 to 6, more preferably 1 to 4, carbon atoms; examples are methyl, ethyl, propyl, (n- or iso-propyl) and butyl (n-, sec-, iso- or t-butyl).

R¹ is C₁₋₄, particularly C₁₋₂, alkyl or C₁₋₄ haloalkyl groups. A preferred substituent is fluorine of which one or more atoms may be present. It is particularly preferred that R¹ is methyl, being optionally substituted by one, two or three fluorine atoms.

Halogen atoms, wherever referred to are particularly fluorine, chlorine or bromine atoms and especially fluorine or chlorine atoms.

Cycloalkyl is C₃₋₆ cycloalkyl, for example cyclohexyl, and cycloalkylalkyl is C₃₋₆ cycloalkyl(C1-4)alkyl, for example, cyclopropylethyl. Alkenyl and alkynyl groups contain 2 to 6, preferably 2 to 4, carbon atoms in the form of straight or branched chains. Examples are ethenyl, allyl and propargyl. Phenyl(C1-4)alkyl is preferably benzyl, phenylethyl or phenyl n-propyl. Optionally substituted alkyl includes in particular, halo-(C₁₋₆)alkyl, optionally substituted phenyl(C₁₋₆)alkyl, and optionally substituted phenoxy(C₁₋₆)alkyl; optionally substituted phenylalkenyl is especially optionally substituted phenylethenyl; and optionally substituted phenyl(C₁₋₆)alkoxy includes optionally substituted benzyloxy. Optional substituents for "alkoxy" and "alkylthio" include those described above for "alkyl".

Substituents which may be present in any optionally substituted phenyl or heteroaryl moiety include one or more of the following; halogen, hydroxy, C₁ -₄ alkyl (especially methyl and ethyl), C₁ -₄ alkoxy (especially methoxy), halo(C₁₋₄)alkyl (especially trifluoromethyl), halo(C₁₋₄)alkoxy (especially trifluoromethoxy), C₁₋₄ alkylthio (especially methylthio), (C₁₋₄)alkoxy(C₁₋₄)alkyl, C₃₋₆ cycloalkyl, C₃₋₆ cycloalkyl(C1-4)alkyl, phenyl, phenyloxy, phenyl(C1-4)alkyl (especially benzyl, phenylethyl and phenyl n-propyl), phenyl(C₁₋₄)alkoxy (especially benzyloxy), phenyloxy(C₁₋₄)alkyl (especially phenyloxymethyl), cyano, thiocyanato, nitro, -NR'R", -NHCOR', -NHCONR'R", -CONR'R", -COOR', -OS0₂R', -S0₂R', -COR', -OCOR', -CR' = NR" or -N = CR'R" in which R' and R" are independently hydrogen, C₁₋₄ alkyl, C₁₋₄ alkoxy, C₁₋₄ alkylthio, C₃₋₆ cycloalkyl, C₃₋₆ cycloalkyl(C₁₋₄)alkyl, phenyl or benzyl, the phenyl and benzyl groups being optionally substituted with halogen, C₁ -₄ alkyl or C₁ -₄ alkoxy.

The substituents on the substituted pyridinyl or substituted pyrimidinyl ring W, which are the same or different, include any of the values given for Y and Z. In particular, they include halogen atoms, or hydroxy, optionally substituted alkyl (including haloalkyl), especially C₁₋₄ alkyl, optionally substituted alkenyl, especially C₃-C₄ alkenyl, optionally substituted phenyl, optionally substituted alkynyl, especially C₃-C₄ alkynyl, optionally substituted alkoxy (including haloalkoxy), especially C₁ -₄ alkoxy, optionally substituted phenyloxy, optionally substituted heterocyclyloxy, (especially heteroaryloxy), optionally substituted heterocyclyl, nitro, cyano, -NR'R", -NHCOR', -CONR'R", -OCOR', -C0₂R', -COR', -CH=NOR", -CH₂NR'R", -CH₂0R', -CH₂NHCOR', -CH₂0COR', or S(O)ₘR' (wherein m is 0, 1 or 2); and R', R", R³, R⁴, R⁵, R⁶ and R⁷ are as defined above.

Pyridines and pyrimidines with hydroxy substituents in appropriate positions may also exist in the corresponding tautomeric oxo-forms, ie. as the corresponding pyridones and pyrimidones, respectively. It is intended that when there is a hydroxy substituent on the pyridinyl or pyrimidinyl ring W the present invention should include all such tautomeric forms and mixtures thereof (see, for example, G.R.Newkome and W.W.Paudler, Contemporary Heterocyclic Chemistry, Wiley - Interscience pp236-241).

Preferred substituent haloalkyl and haloalkoxy groups are halo(C₁₋₄ )alkyl and halo(C₁₋₄)alkoxy groups. Haloalkyl includes particularly trihalomethyl and especially trifluoromethyl (except where otherwise indicated).

Substituents on a substituted amino group, or moiety are preferably C₁ -₄ alkyl.

Heterocyclic groups, or moieties (e.g. as in heterocyclyl or heterocyclyloxy) are, 2-, 3-, or 4-optionally substituted pyridines or 2-, 4- or 5- optionally substituted pyrimidines.

In one particular aspect, the invention provides compounds having the formula (XV): 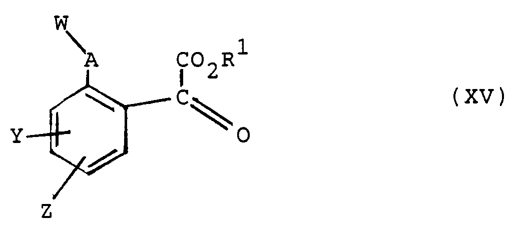 wherein W is a substituted pyridinyl or a substituted pyrimidinyl group linked to A by any one of its carbon atoms and bearing one or more substituents selected from halogen atoms, C₁ -₆ alkyl itself optionally substituted with halo, phenyl or phenoxy, C₂-₆ alkenyl, phenyl(C₂-₆)alkenyl, C₂-₆ alkynyl, C₁₋₆ alkoxy itself optionally substituted with halo, phenyl or phenoxy, phenoxy, pyridinyloxy, pyrimidinyloxy, phenyl, pyridinyl, pyrimidinyl, nitro, cyano, -NR'R", -NHCOR', -CONR'R", -OCOR', -C0₂R', -COR' or S(O)ₘR' groups, wherein m is 0, 1 or 2 and R' and R" are as defined above; A is either an oxygen atom or S(O)ₙ wherein n is 0, 1 or 2; Y and Z, which are the same or different, are hydrogen, fluorine, chlorine or bromine atoms, or C₁₋₄ alkyl (optionally substituted with halogen, C₁₋₄ alkoxy or phenyl) , C₂-₅ alkenyl, C₂-₅ alkynyl, phenyl, C₁₋₄ alkoxy (optionally substituted with halogen, C₁₋₄ alkoxy or phenyl), phenoxy, benzyloxy or mono- or di(Cᵢ-₆)alkylamino groups; wherein the phenyl moieties of Y and Z and of the substituents of W of any of the foregoing are optionally substituted with one or more fluorine, chlorine or bromine atoms, or phenyl, C₁₋₄ alkyl, C₁₋₄ alkoxy, nitro, amino, cyano, hydroxyl or carboxyl groups; and R¹ is C₁ -₄ alkyl (especially methyl) optionally substituted with one, two or three halogen (especially fluorine) atoms; provided that when W is 5-trifluoromethylpyridin-2-yl, A is oxygen and R¹ is methyl, Y and Z are not both hydrogen, Y is not F, Cl, methyl, nitro, 5-CF₃ or 4-(CH₃)₂N if Z is hydrogen and Y and Z together are not 3-nitro-5-chloro, 3,5-dinitro or 4,5-dimethoxy.

When one or both of Y and Z are other than hydrogen it is preferred that they are single atoms or sterically small groups such as fluorine, chlorine, bromine, hydroxy, methyl, methoxy, trifluoromethyl, methylamino and dimethylamino. It is further preferred that one of such substituents occupies the 5-position of the phenyl ring, especially a single substituent such as chlorine.

In another aspect the invention provides compounds having the formula (XVa): 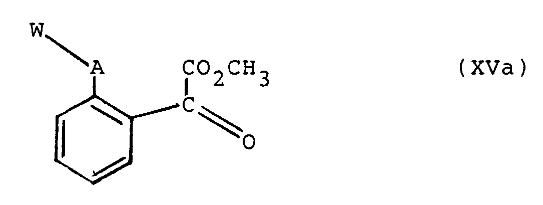 wherein A is S(O)ₙ wherein n is 0, 1 or 2, or preferably, an oxygen atom; W is a substituted pyridinyl or a substituted pyrimidinyl group linked to A by any one of its carbon atoms, the substituents on the pyridinyl or pyrimidinyl rings, which are the same or different, being one or more halogen atoms or C_{1 -6} alkyl itself optionally substituted with halo, phenyl or phenoxy, C₂-₆ alkenyl, phenyl(C₂-₆)alkenyl, C₂-₆ alkynyl, alkoxy itself optionally substituted with halo, phenyl or phenoxy, phenoxy, pyridinyloxy, pyrimidinyloxy, phenyl, pyridinyl, pyrimidinyl, -OCOR', -NR'R", -NHCOR' wherein R' and R" are as defined below, nitro, cyano, -C02 R3, -CONR4R5, -COR⁶ or S(O)ₘR⁷ groups, wherein m is 0, 1 or 2; R³, R⁴, R⁵, R⁶ and R⁷, which are the same or different, are hydrogen atoms or C₁₋₆ alkyl, C₃₋₆ cycloalkyl, C₃₋₆ cycloalkyl(C_{1 -4})alkyl, C2-6 alkenyl, C₂-₆ alkynyl, phenyl or phenyl(C₁₋₆)alkyl; R' and R" are independently hydrogen, C₁₋₄ alkyl, C₁₋₄ alkoxy, C₁₋₄ alkylthio, C₃₋₆ cycloalkyl, C₃₋₆ cycloalkyl(C₁₋₄)alkyl, phenyl or benzyl, in which the phenyl and benzyl groups are optionally substituted with halogen, C₁ -₄ alkyl or C₁ -₄ alkoxy; and wherein any of the foregoing phenyl or heteroaryl moieties of the substituents of W, except where otherwise stated for R' and R", are optionally substituted by one or more of the following: halogen, hydroxy, C₁₋₄ alkyl, C₁₋₄ alkoxy, halo(C₁₋₄)alkyl, halo(C₁₋₄)alkoxy, C₁₋₄ alkylthio, C₁₋₄ alkoxy(C₁₋₄)alkyl, C₃₋₆ cycloalkyl, C₃₋₆ cycloalkyl(C1-4)alkyl, phenyl, phenoxy, phenyl(C1-4)alkyl, phenyl(C₁₋₄)alkoxy, phenoxy(C₁₋₄)alkyl, cyano, thiocyanato, nitro, -NR'R", -NHCOR', -NHCONR'R", -CONR'R", -COOR', -OS0₂R', -S0₂R', -COR', -OCOR', -CR' = NR" or -N = CR'R" wherein R' and R" are as defined above; provided that when A is oxygen, W is not 5-trifluoromethylpyridin-2-yl.

Preferred substituents on the pyridinyl or pyrimidinyl ring are chlorine, fluorine, bromine, methyl, trifluoromethyl (except where otherwise indicated), trichloromethyl and methoxy.

In a still further aspect the invention provides compounds having the formula (XVb): 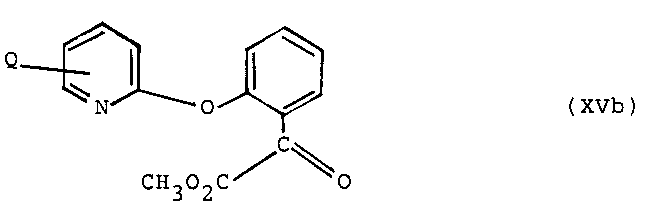 where Q is methyl, trifluoromethyl (but not 5-trifluoromethyl), methoxy, bromine, fluorine or, especially, chlorine.

Q is preferably in the 4-, 5- or 6- position of the pyridine ring, and more preferably in the 4- position when it is methyl, for instance.

The compounds presented in Tables I to III below may be prepared from the compounds of the invention.

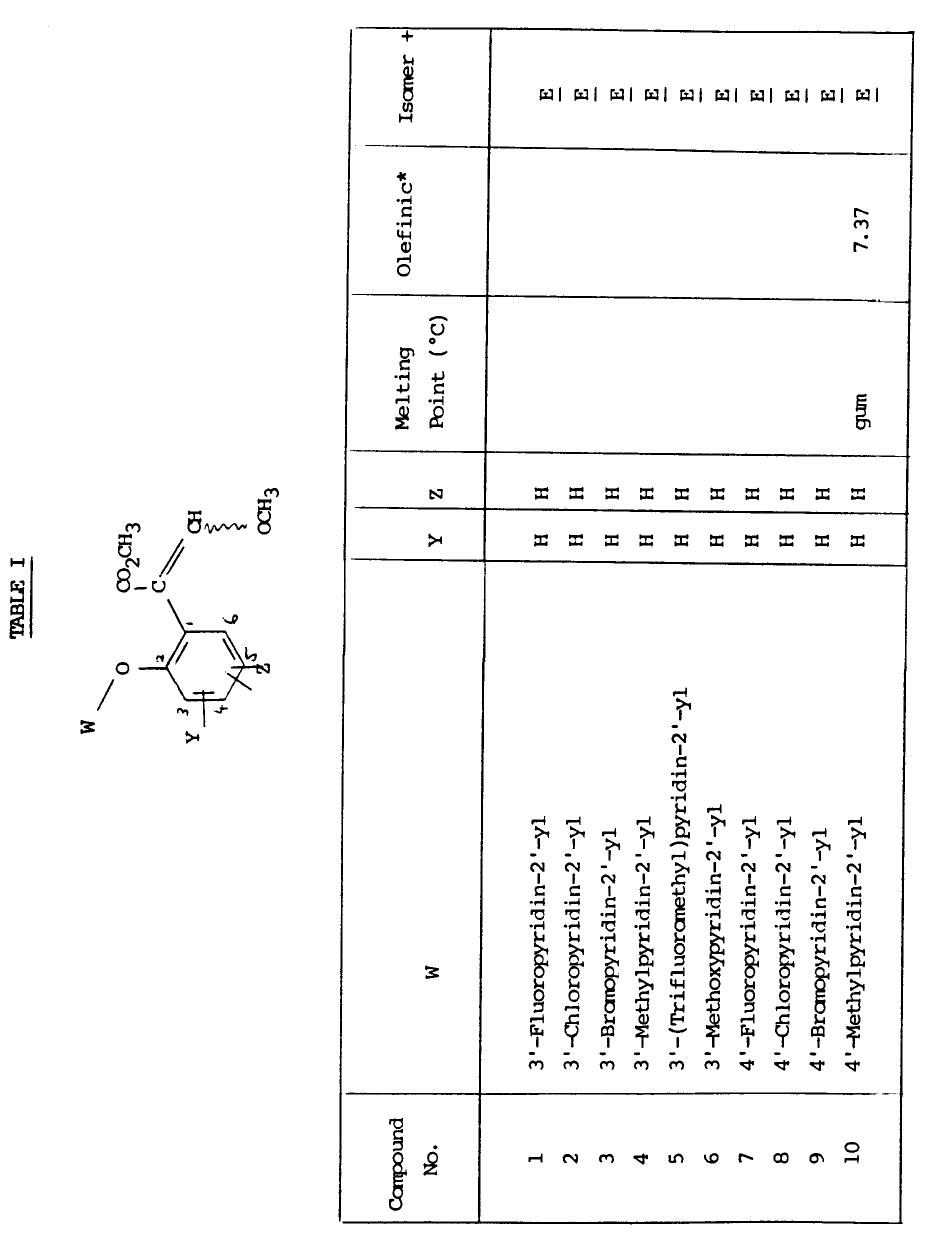

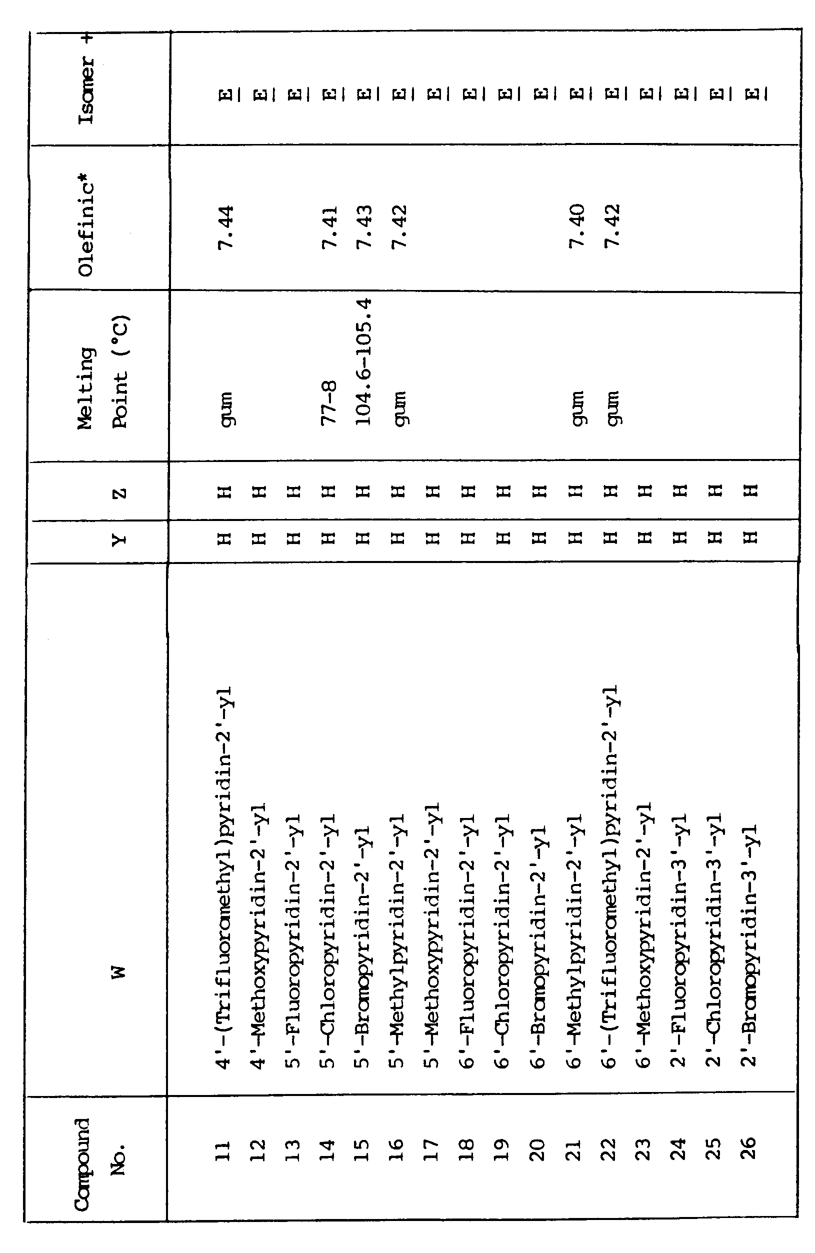

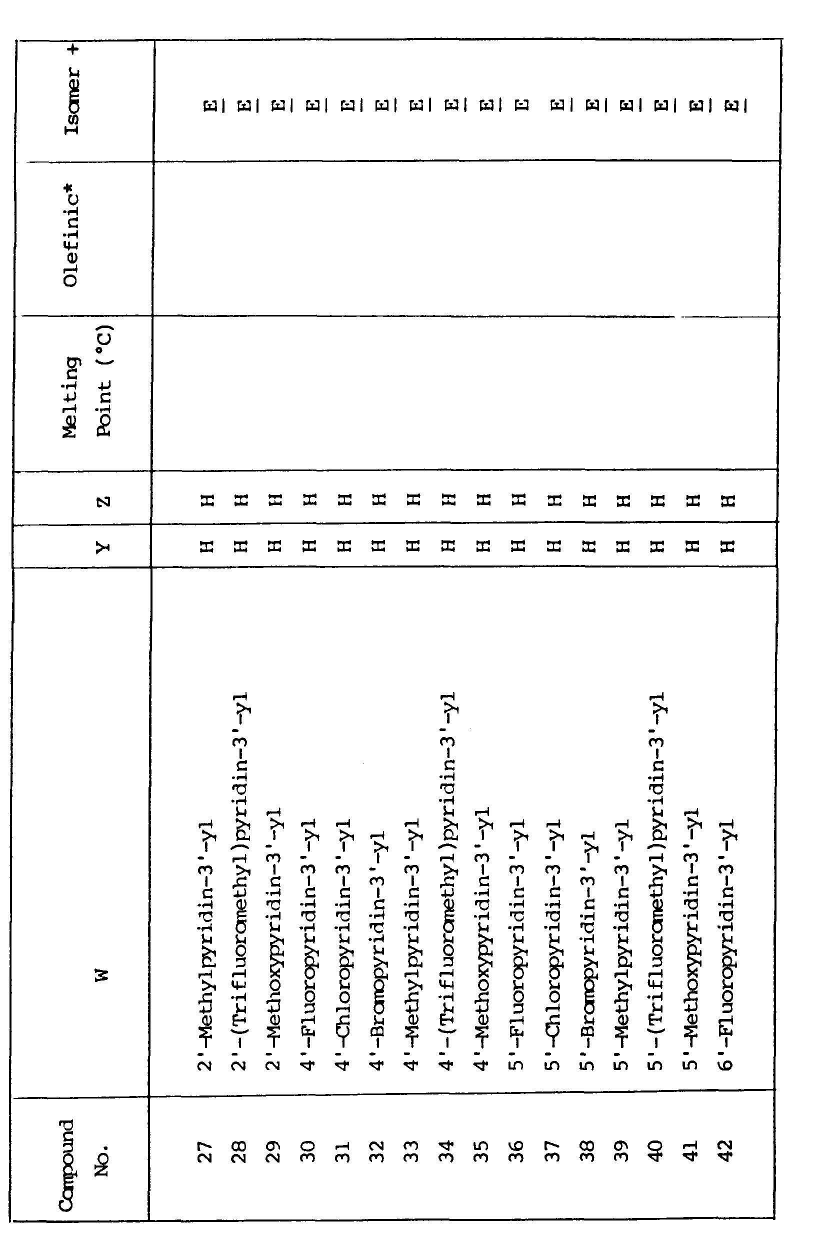

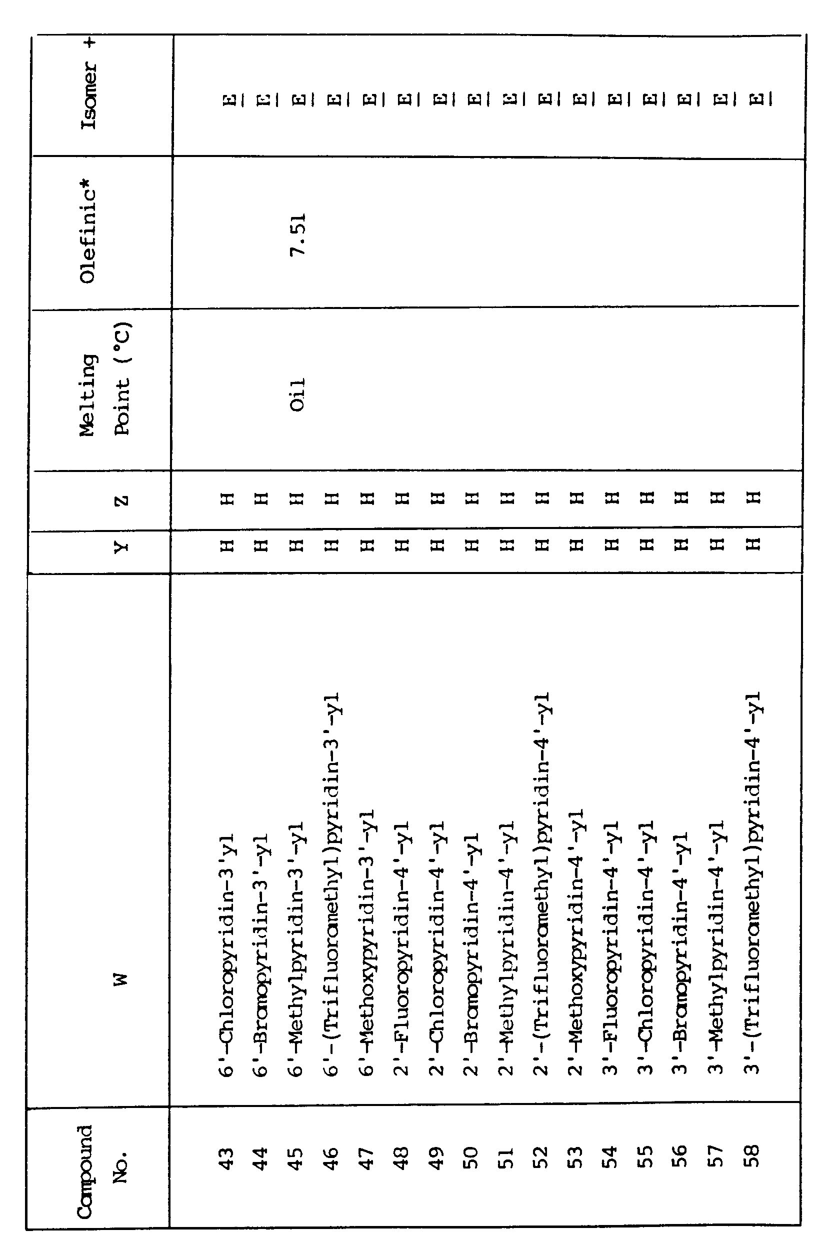

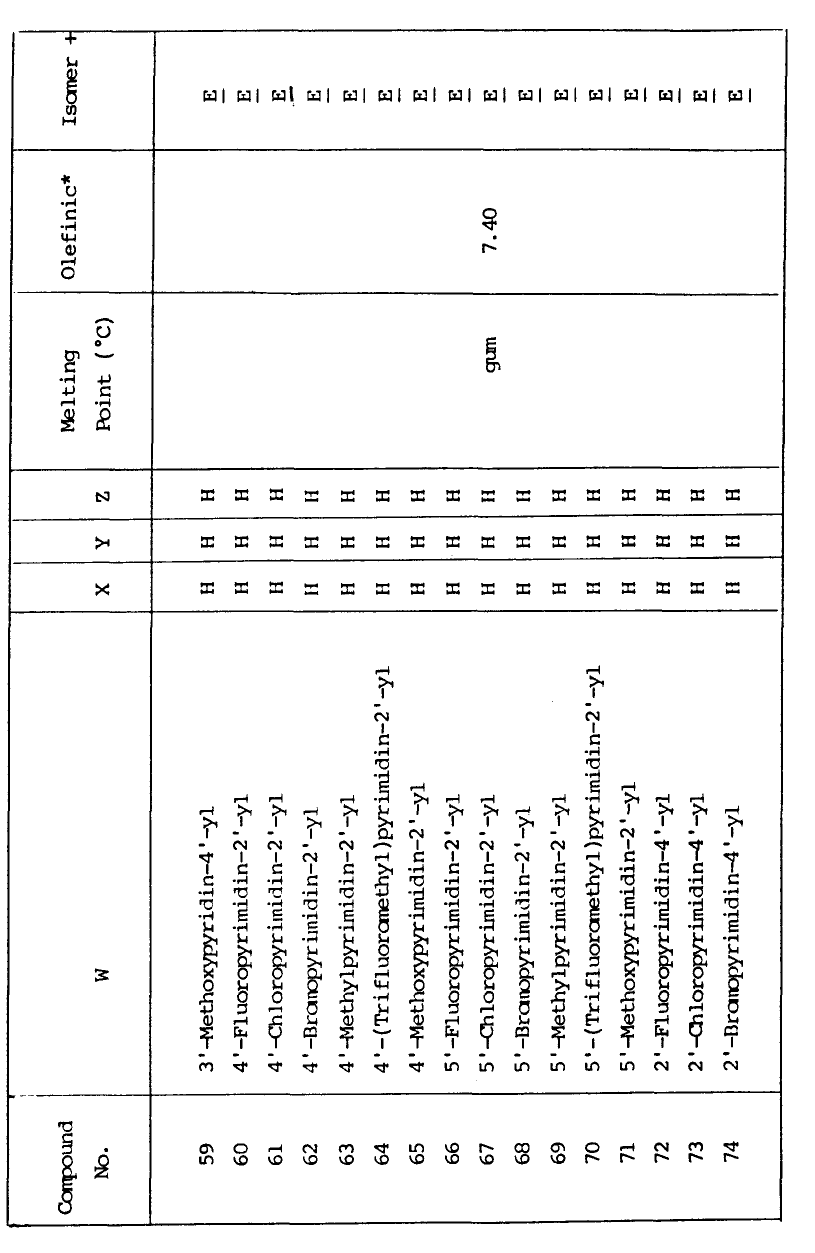

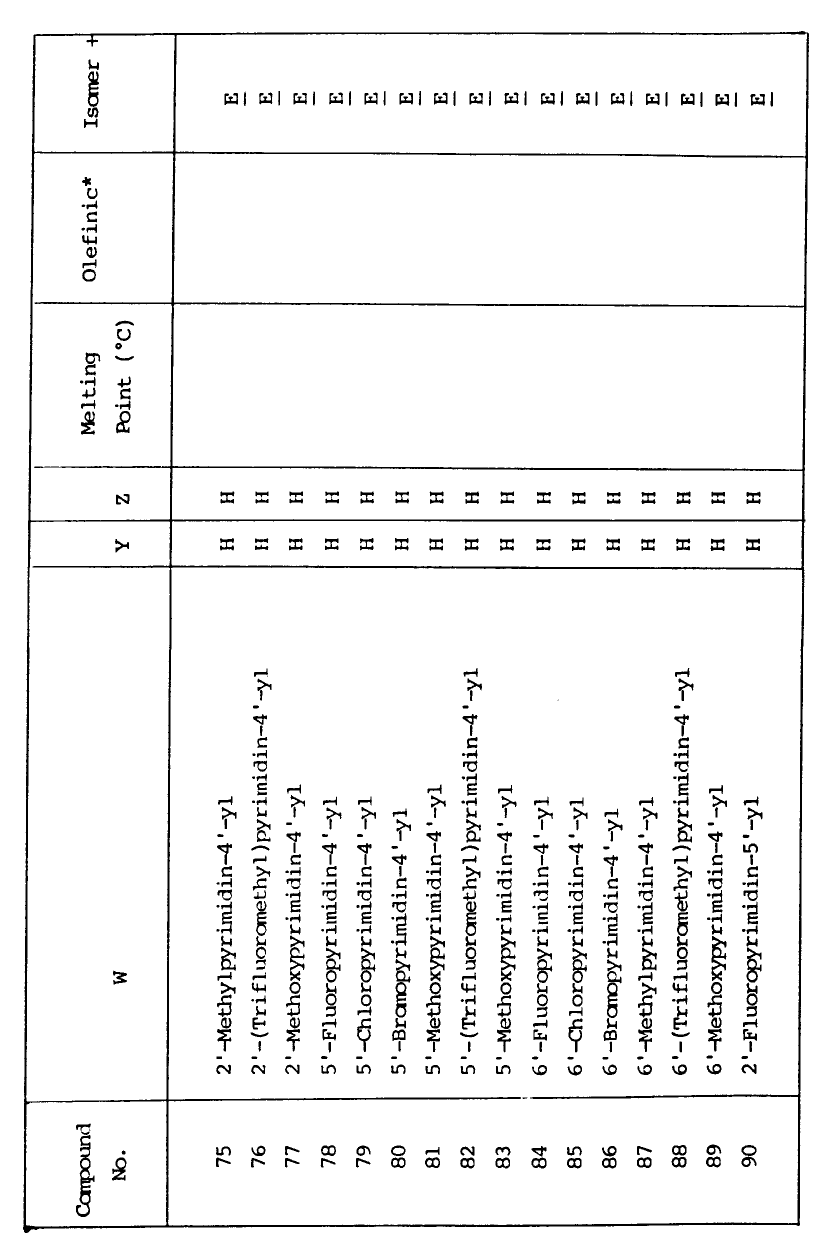

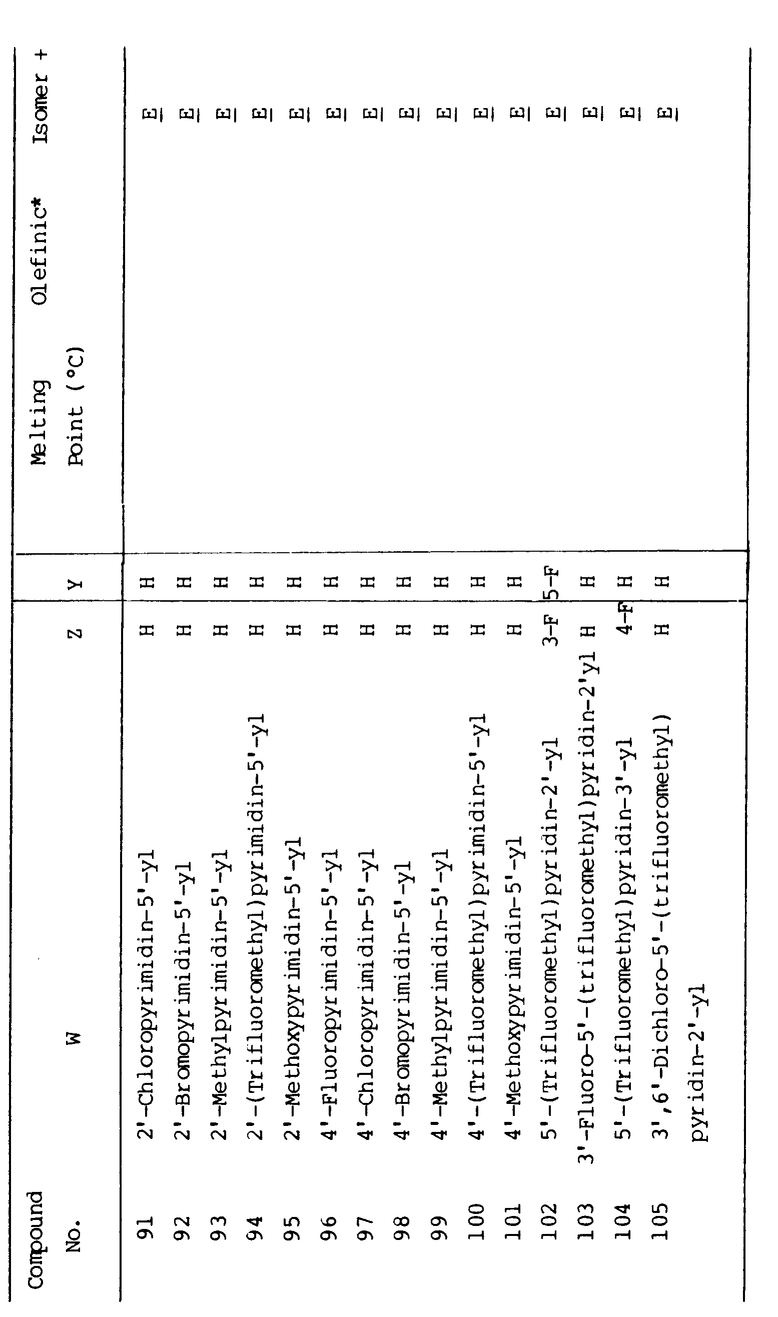

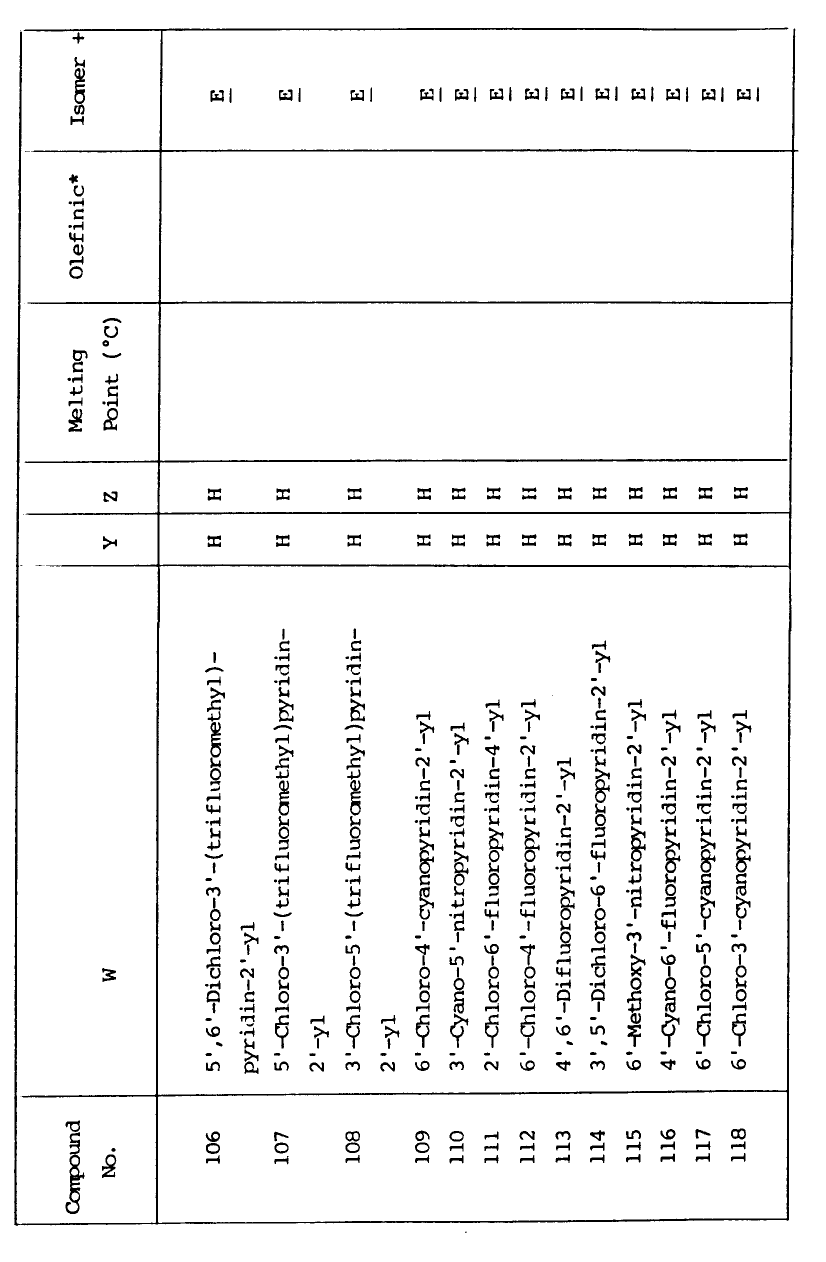

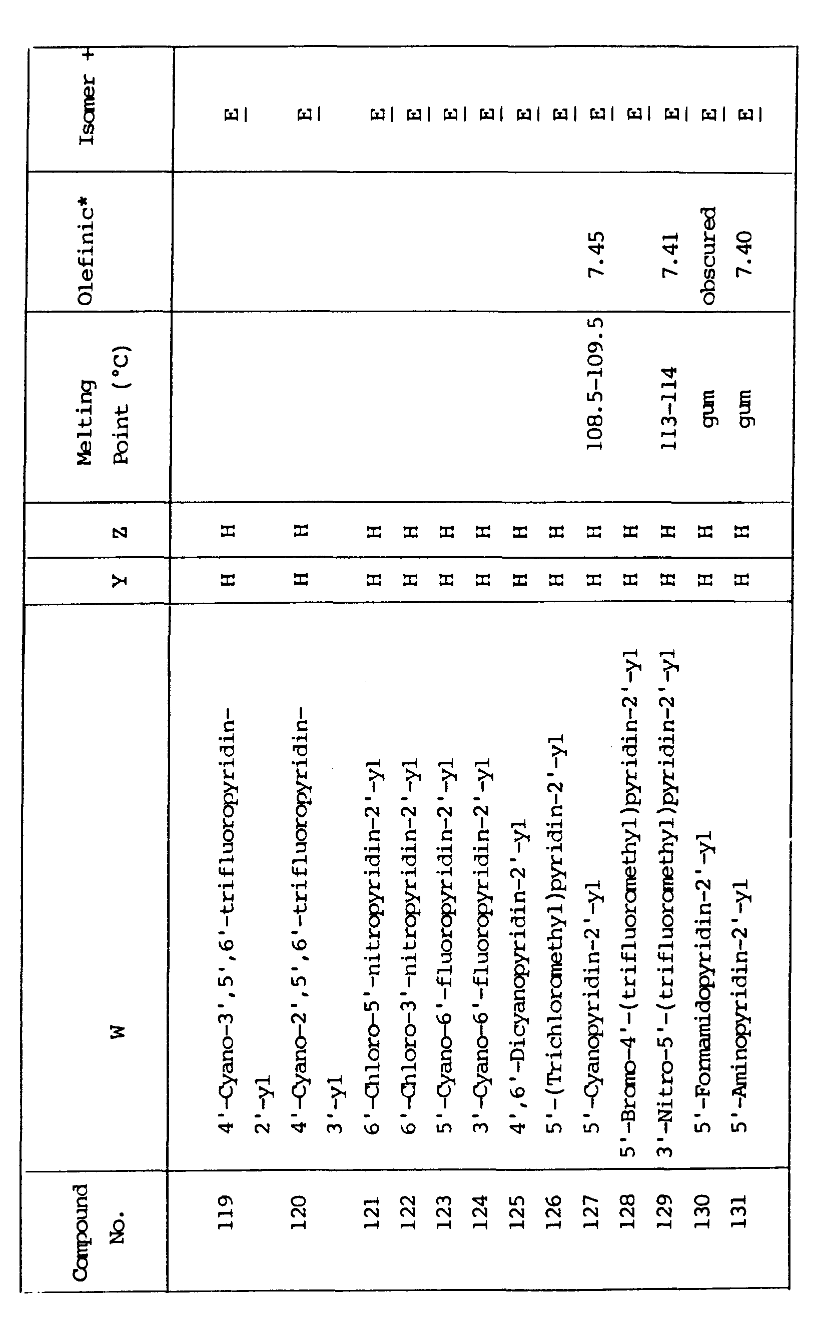

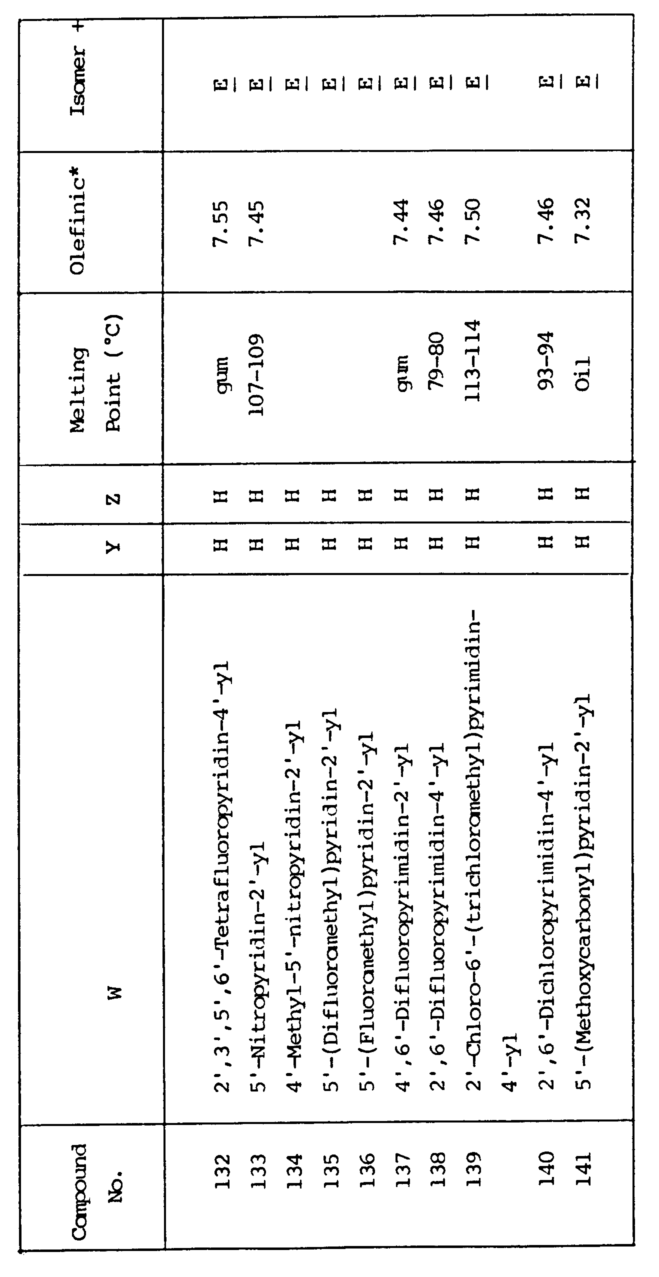

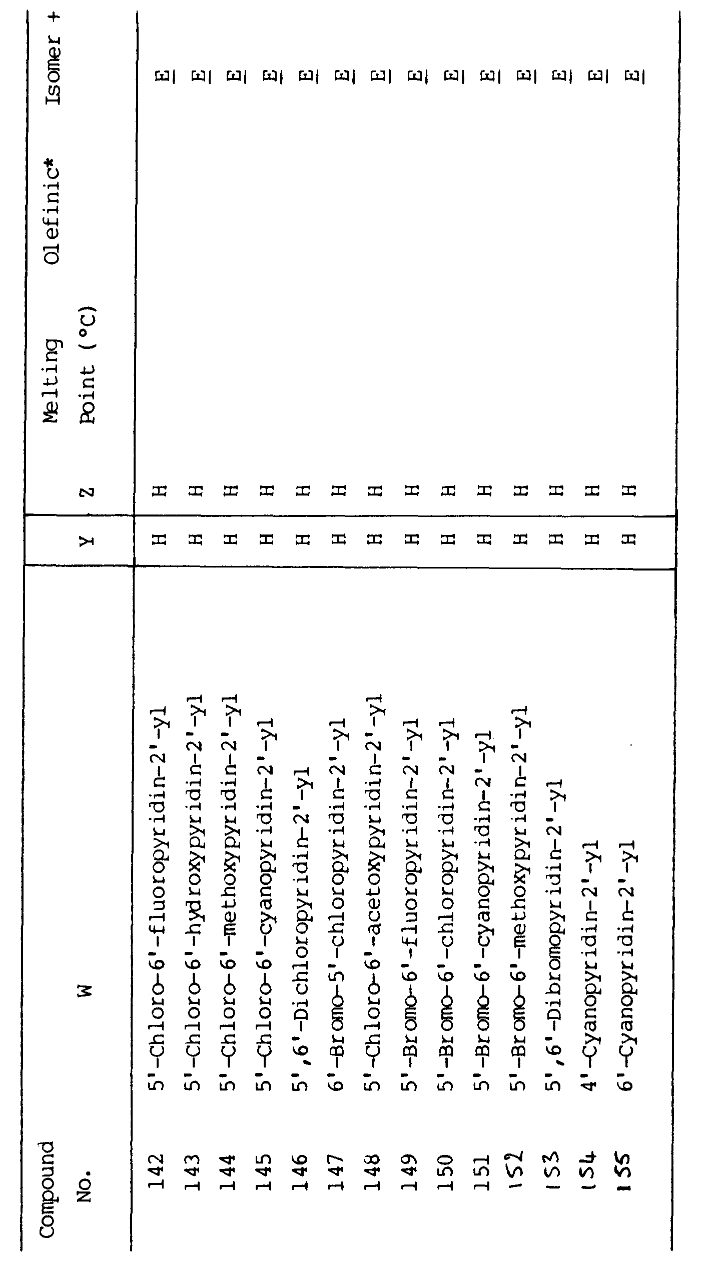

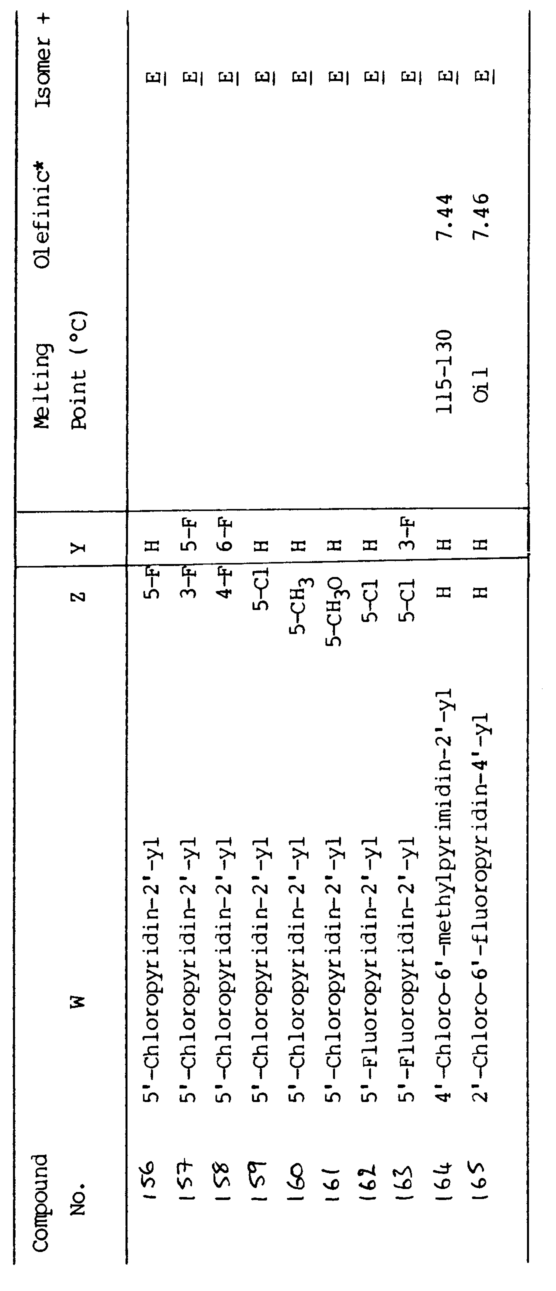

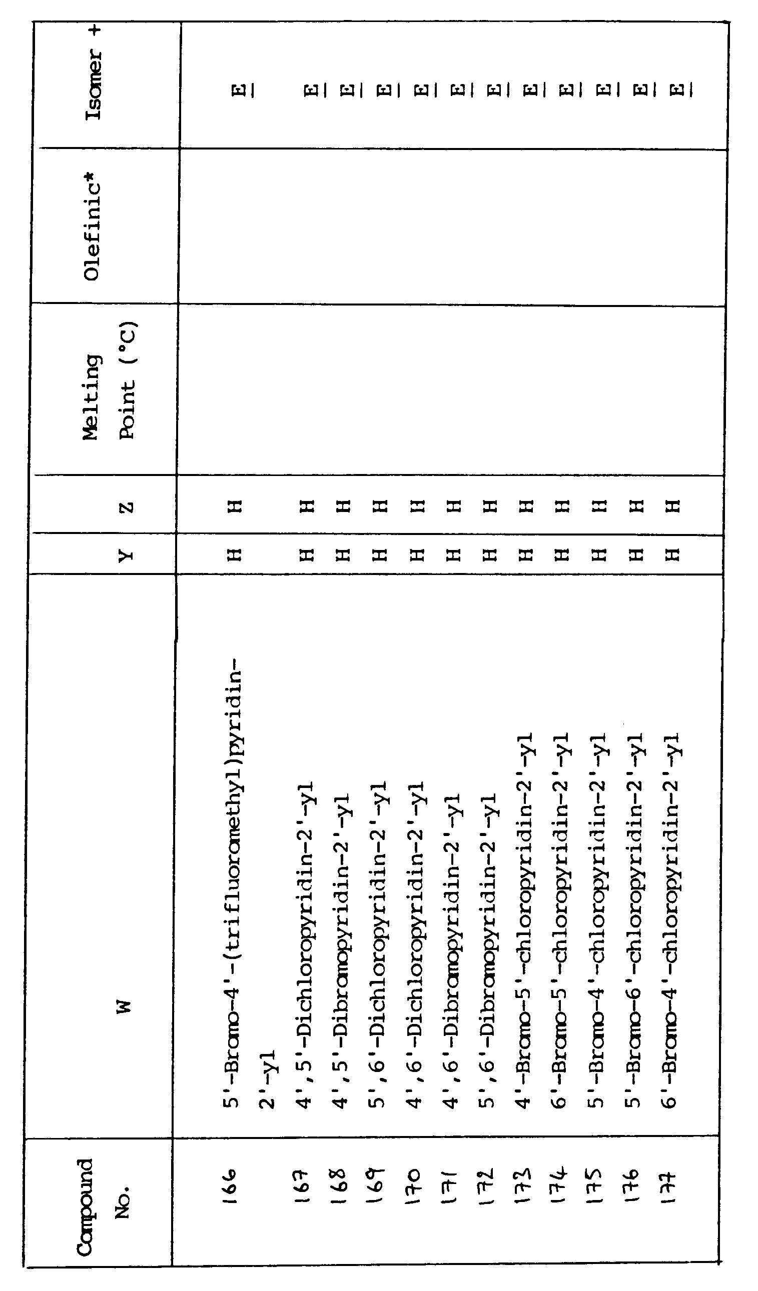

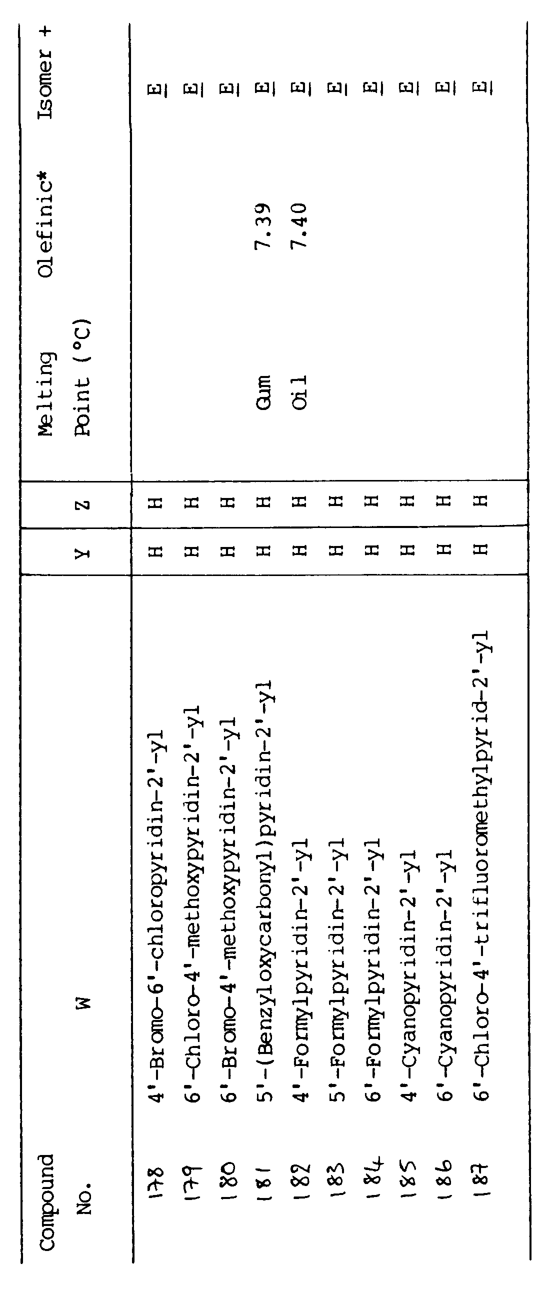

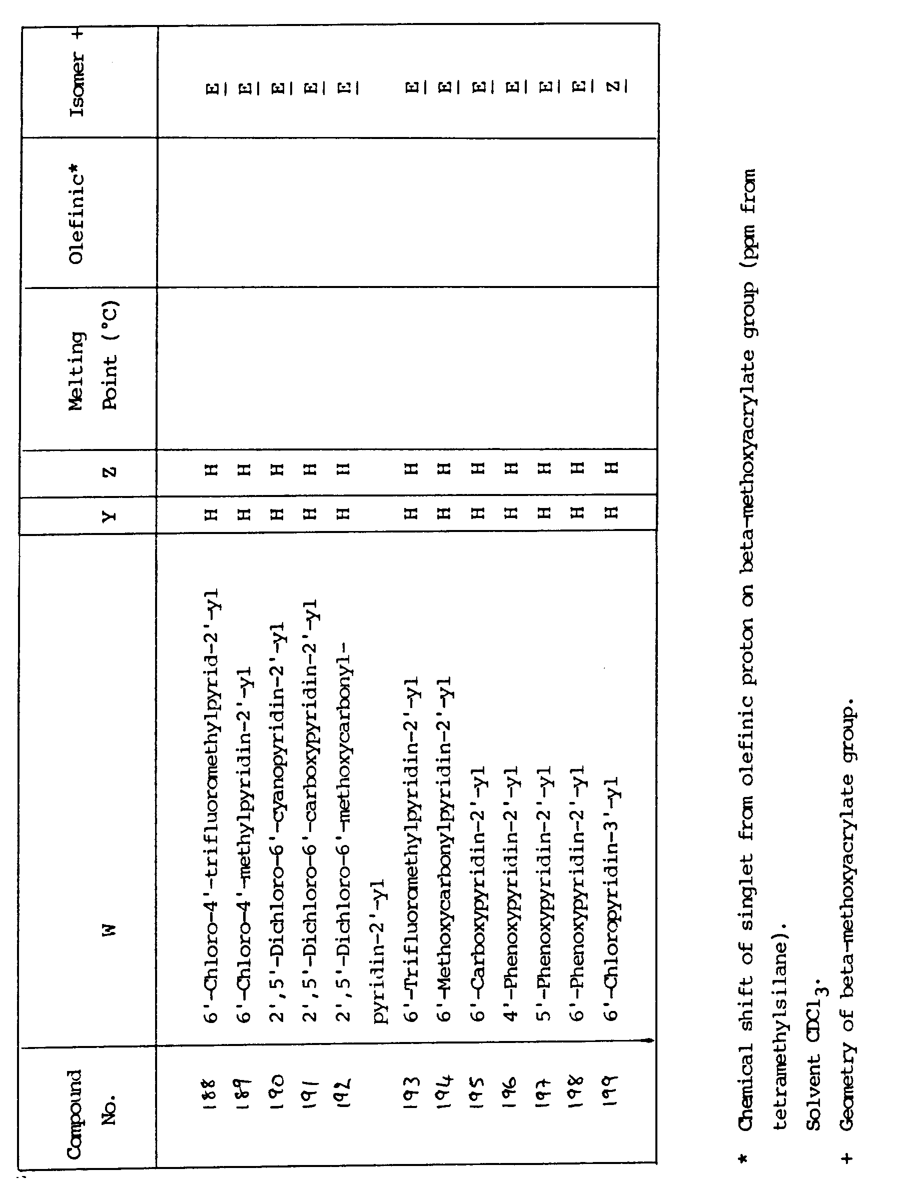

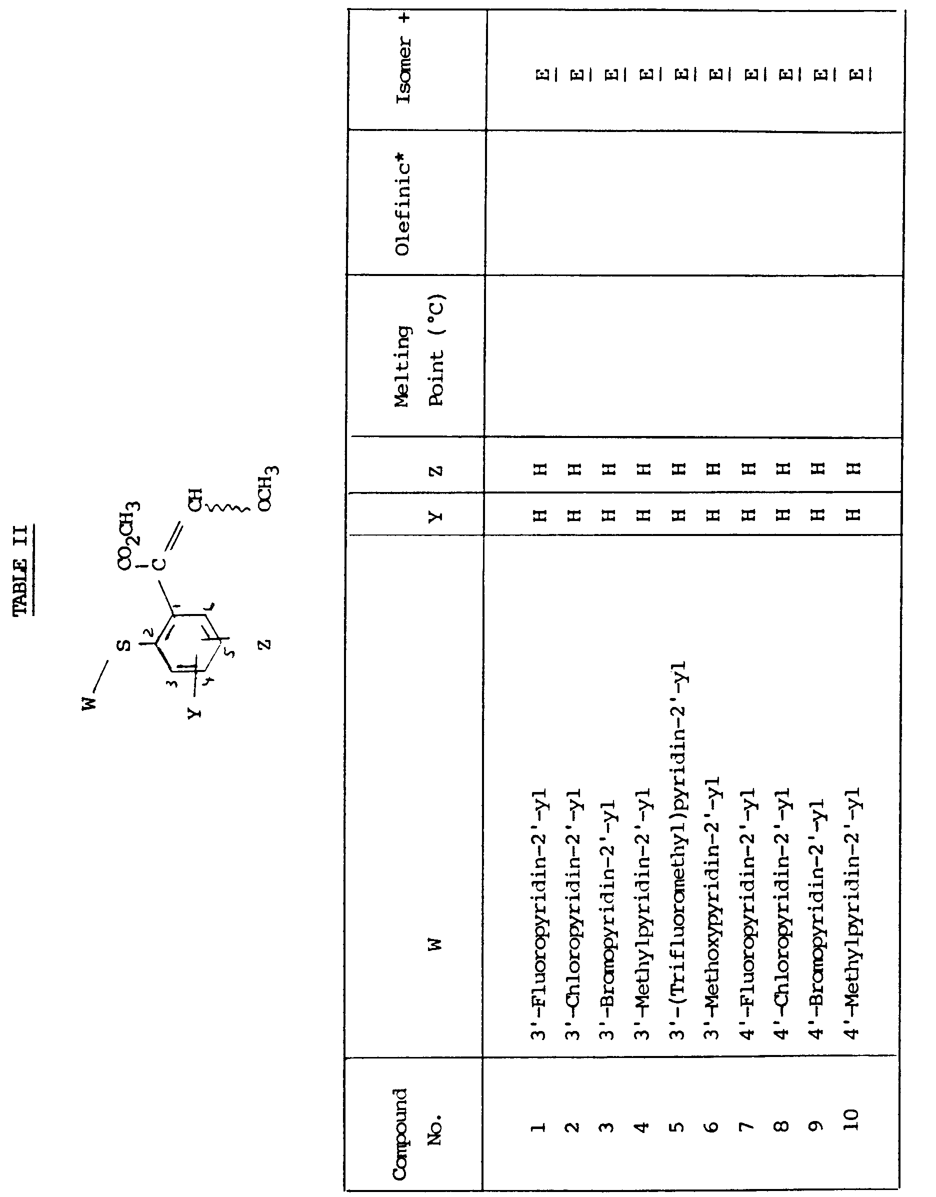

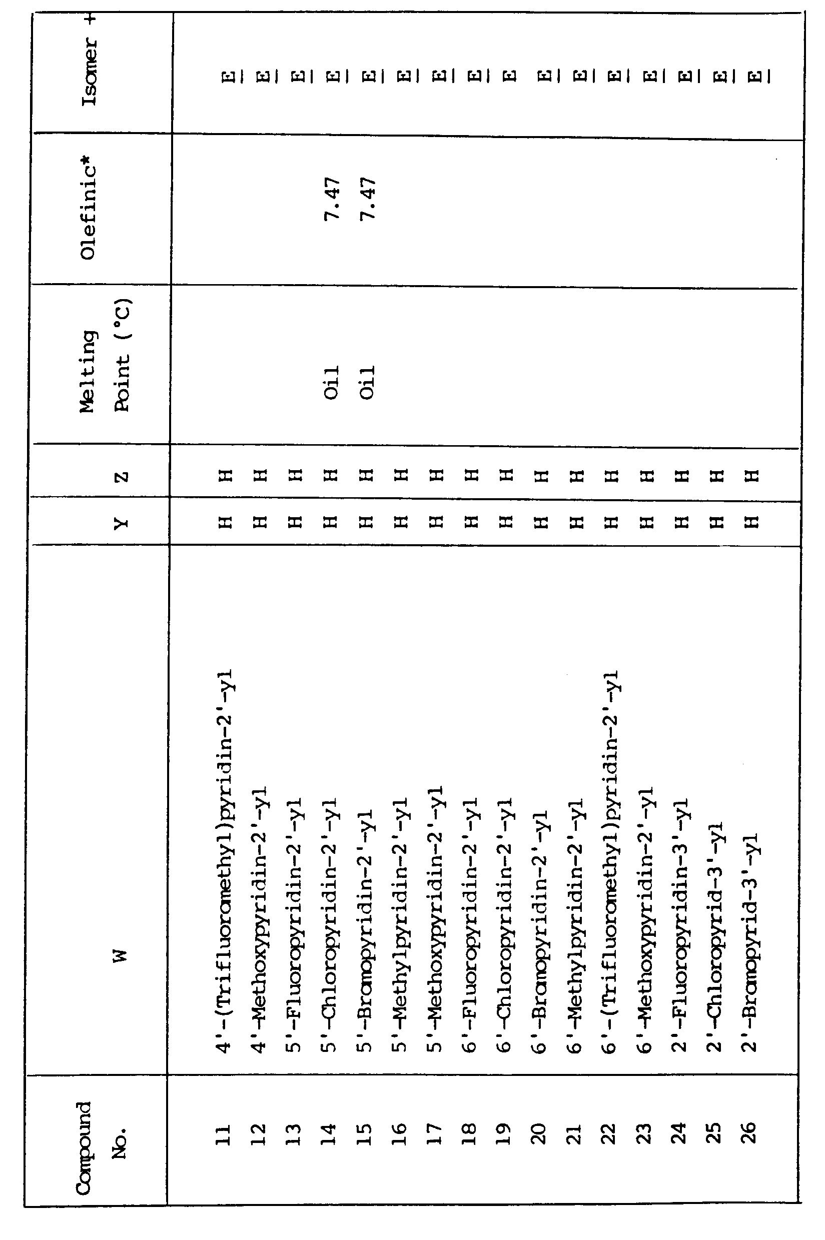

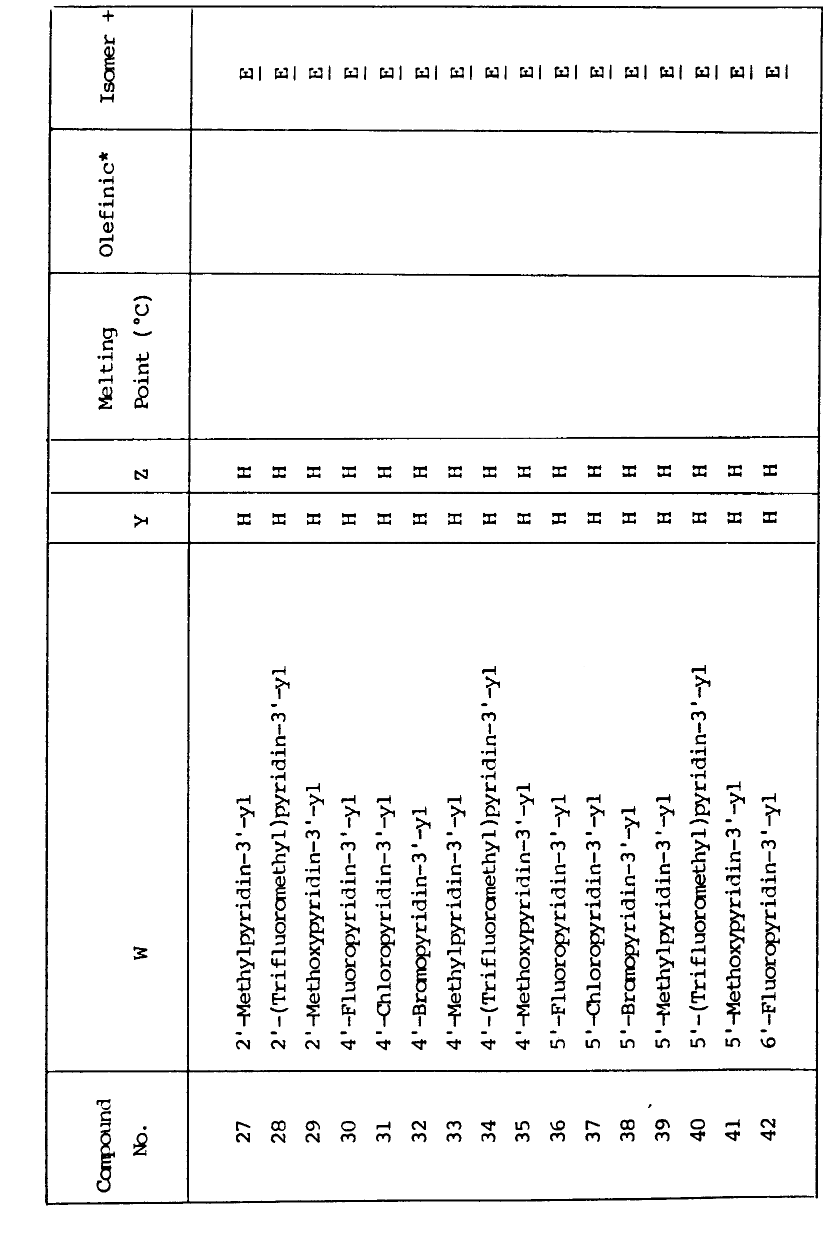

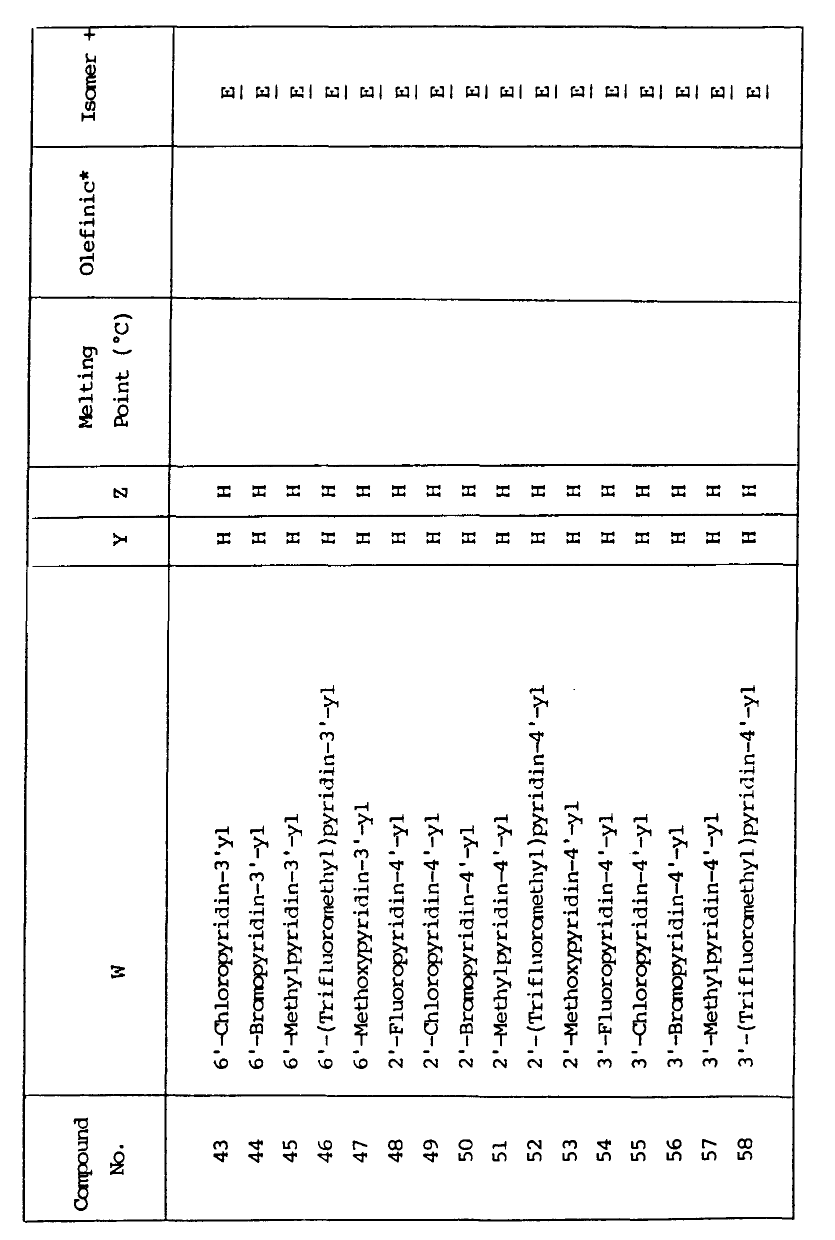

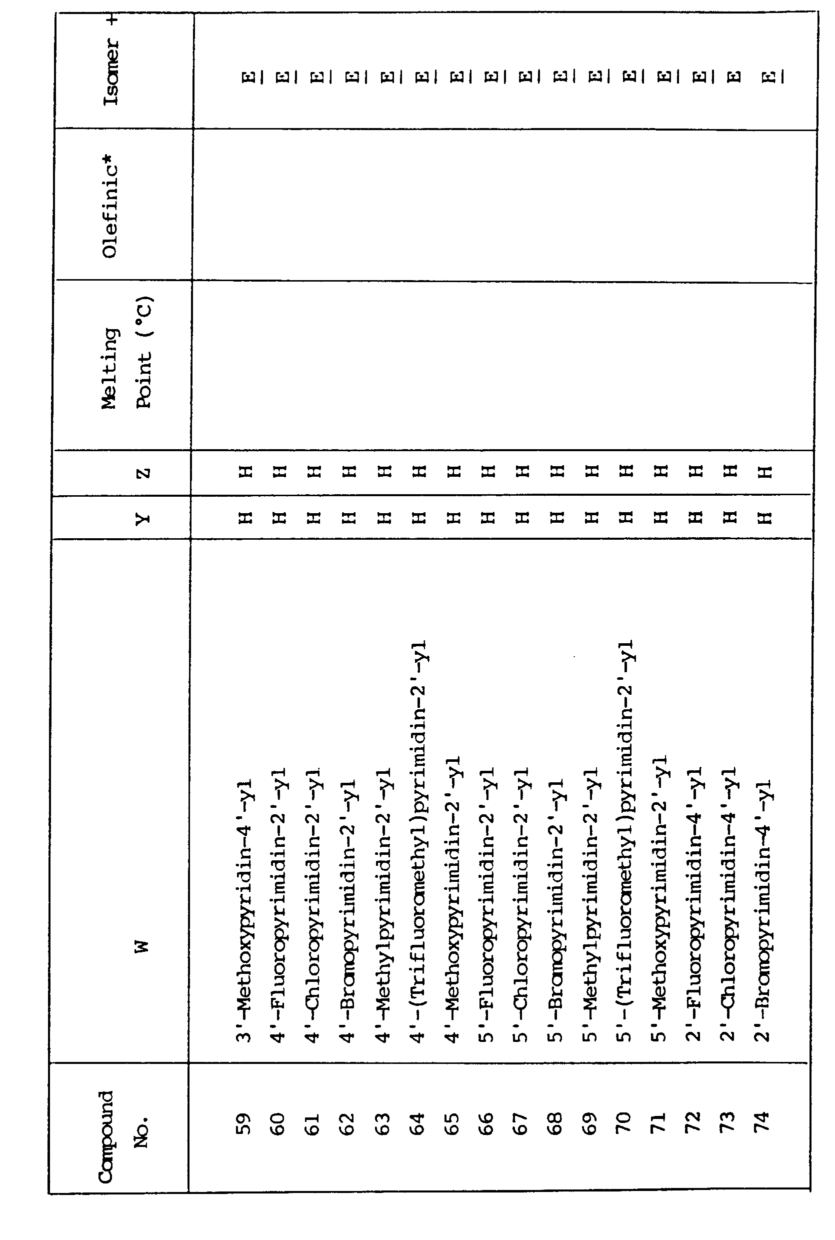

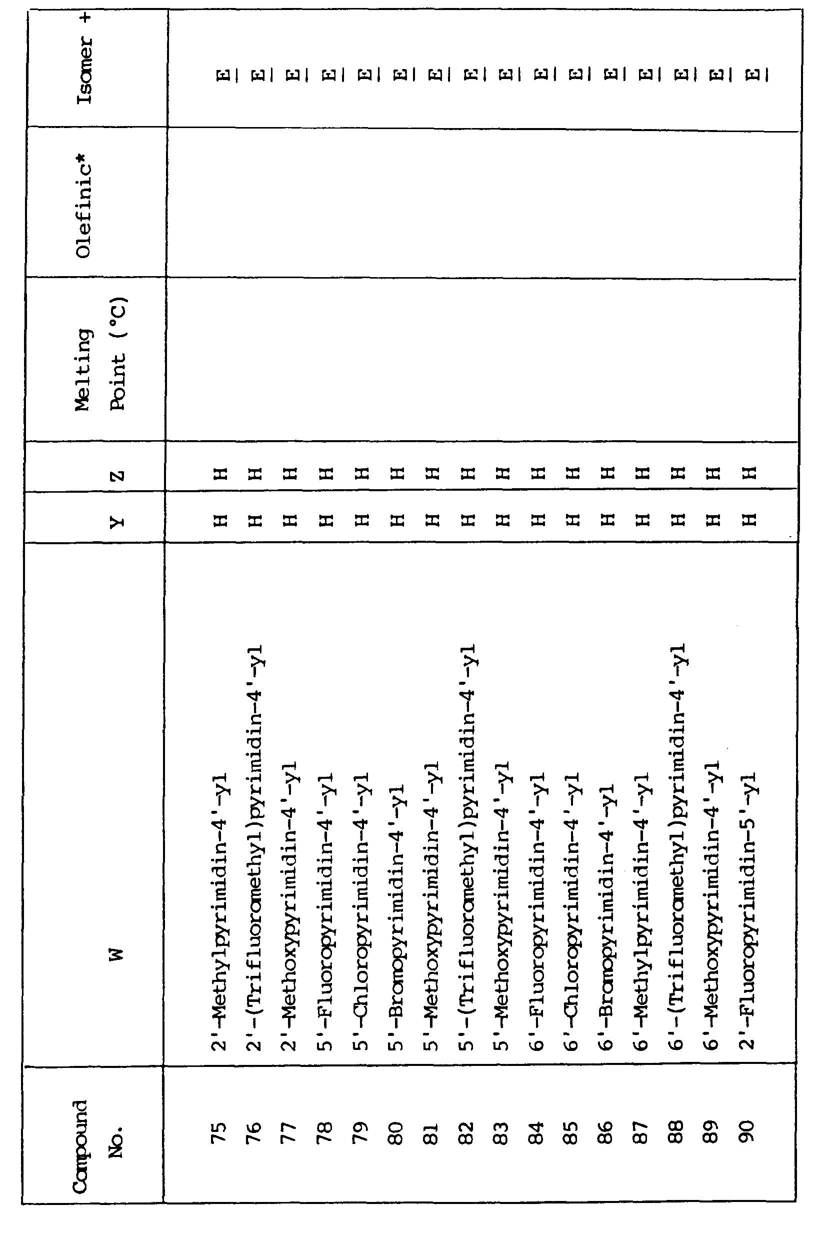

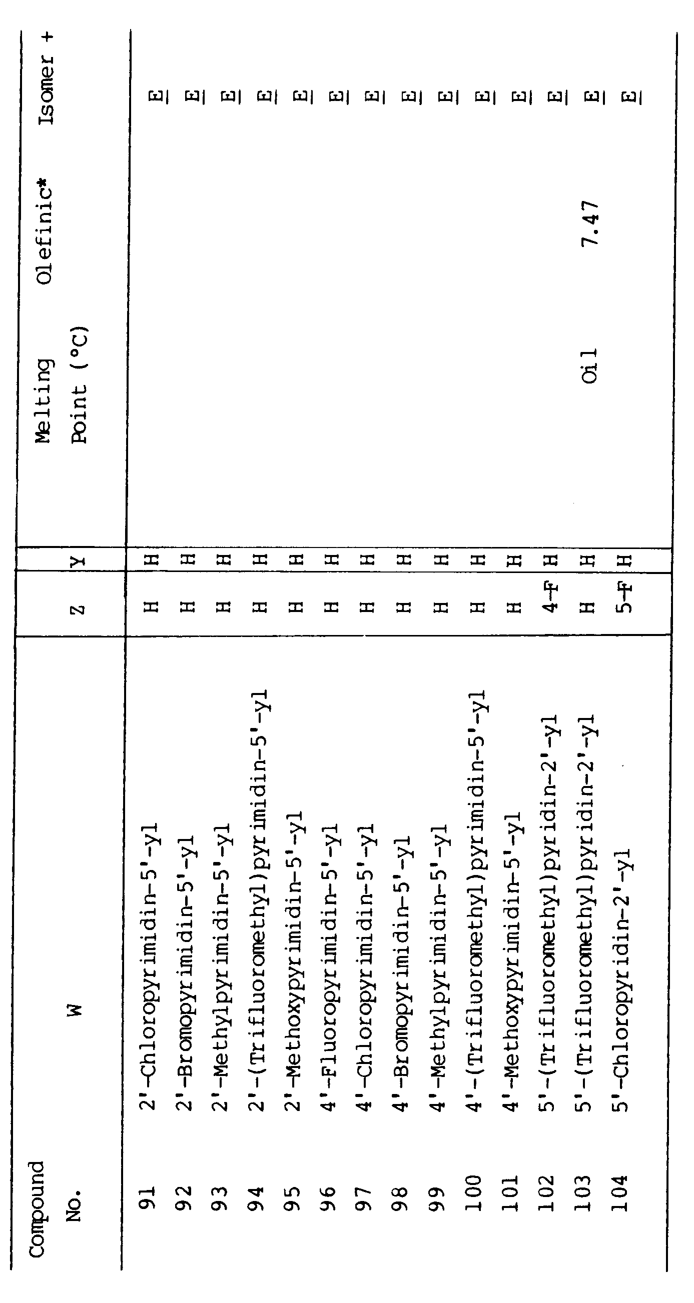

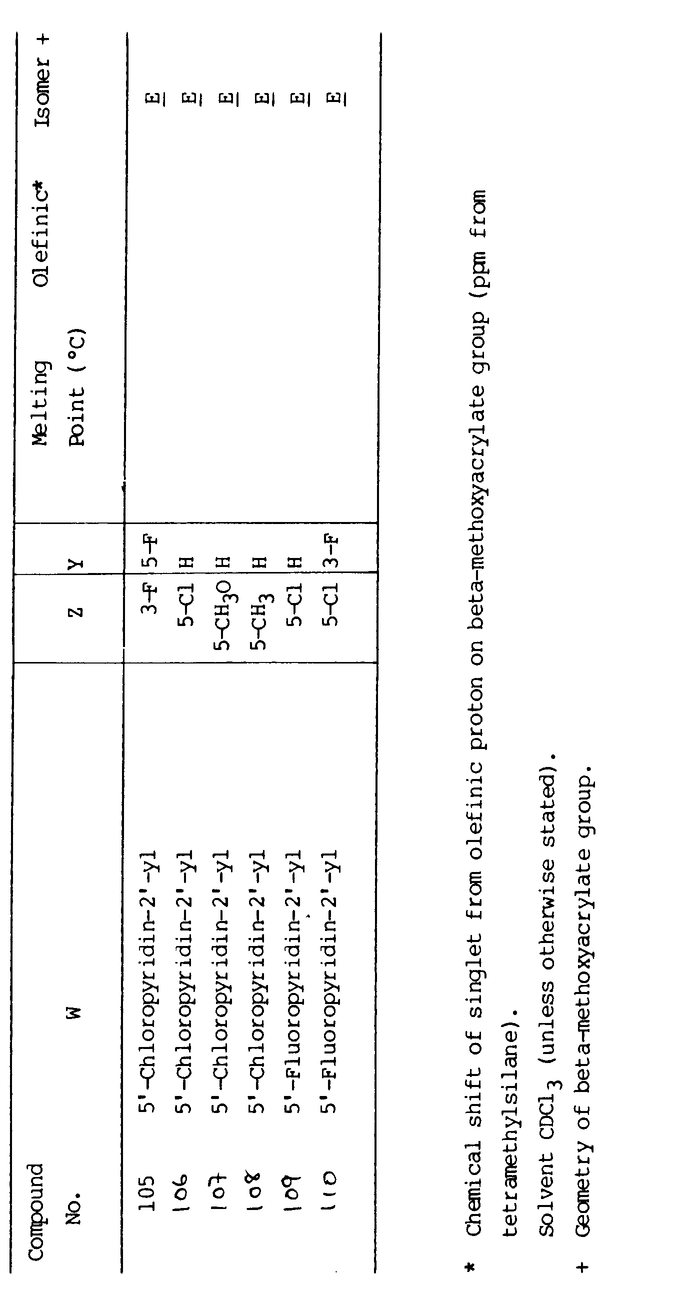

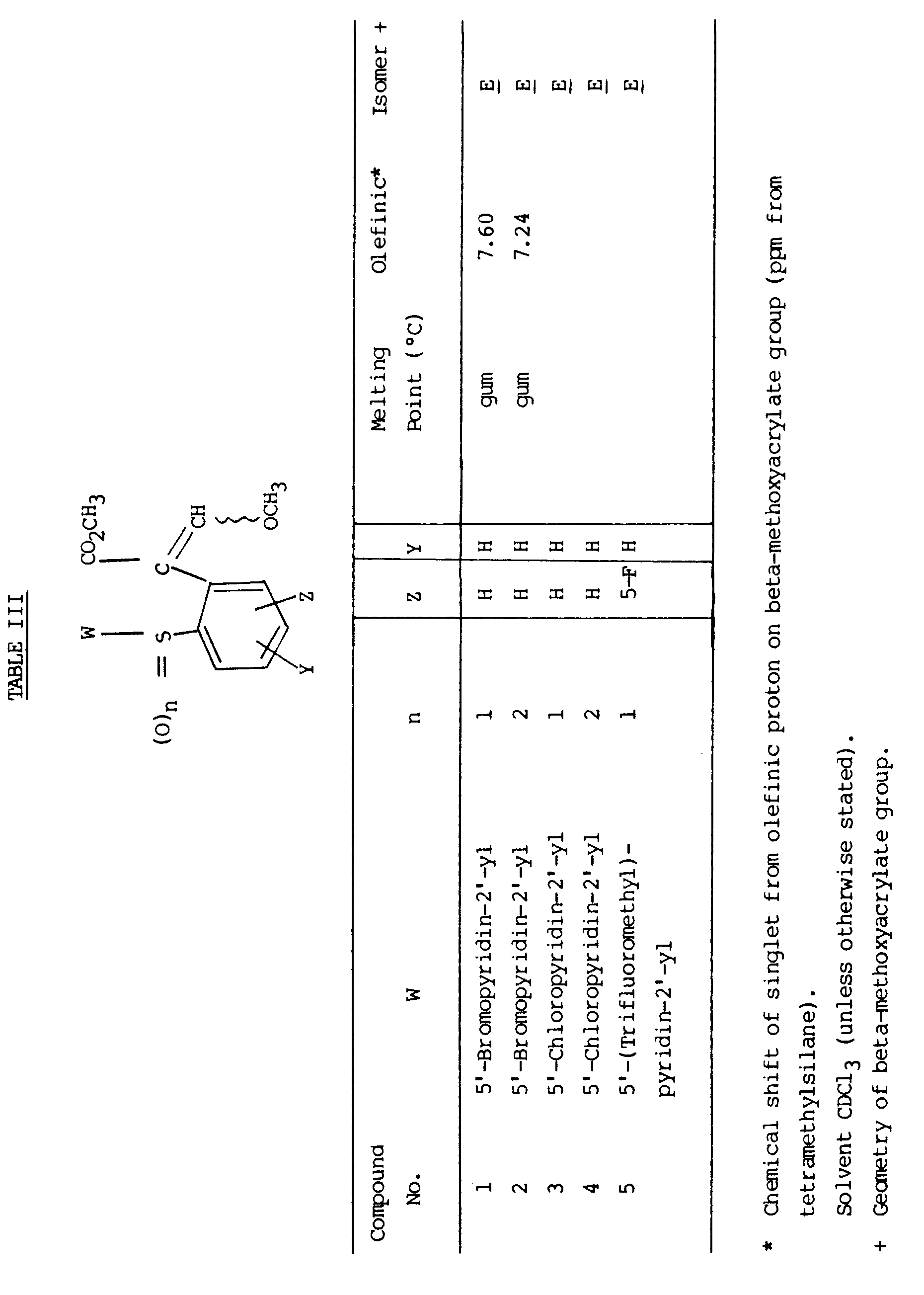

The compounds of the invention having the general formula (XV) may be used to prepare the acrylic acid derivatives of formula (I) as outlined in Scheme III. Throughout Scheme III the terms R¹, R², A, W, Y and Z are as defined above, D is hydrogen or halogen and E is lithium, Mgl, MgBr or MgCI.

Compounds of general formula (I) exist as geometric isomers which may be separated by chromatography, fractional crystallisation or distillation.

Compounds of general formula (I) can be prepared by treatment of ketoesters of general formula (XV) with phosphoranes of general formula (XVI) in a convenient solvent such as diethyl ether (see, for example, EP-A-0044448 and EP-A-0178826) (Step (I) of Scheme III).

Ketoesters of general formula (XV) can be prepared by treatment of metallated species (XVII) with an oxalate (XVIII) in a suitable solvent such as diethyl ether or tetrahydrofuran. The preferred method often involves slow addition of a solution of the metallated species (XVII) to a stirred solution of an excess of the oxalate (XVIII) (see, for example, L.M.Weinstock, R.B.Currie and A.V.Lovell, Synthetic Communications, 1981, 11, 943, and references therein) (step (m) of Scheme III).

The metallated species (XVII) in which E is Mgl, MgBr or MgCl (Grignard reagents) can be prepared by standard methods from the corresponding aromatic halides (XIX) in which D is I, Br or CI respectively. With certain substituents Y and Z, the metallated species (XVII) in which E is Li can be prepared by direct lithiation of compounds (XIX) in which D is H using a strong lithium base such as n-butyl-lithium or lithium di-isopropylamide (see, for example, H.W Gschwend and H. R.Rodriguez, Organic Reactions, 1979, 26, 1) (step (n) of Scheme III).

Compounds of general formula (XIX) can be prepared by standard methods described in the chemical literature.

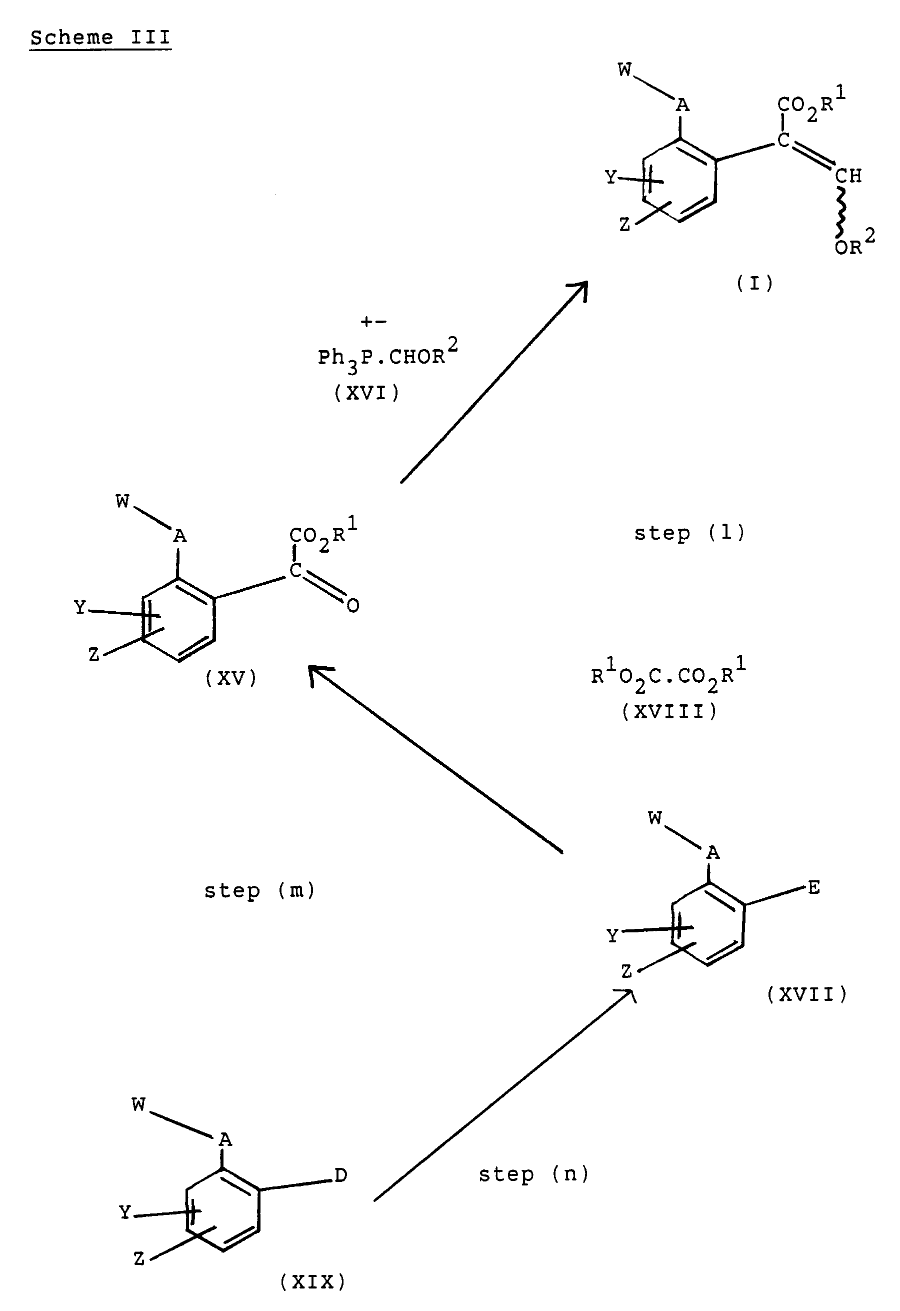

Alternative methods for the preparation of ketoesters of general formula (XV) are described in the chemical literature (see, for example, D.C.Atkinson, K.E.Godfrey, B.Meek, J.F. Saville and M.R.Stillings, J. Med. Chem., 1983, 26, 1353; D Home, J.Gaudino and W.J.Thompson, Tetrahedron Lett., 1984, 25, 3529; and G.P.Axiotis, Tetrahedron Lett., 1981, 22, 1509).

The acrylic acid derivatives of formula (I), prepared from compounds of the invention, are active as fungicides (see European Patent Application No. 87302795.7 for details).

## Claims (Claims for the following Contracting State(s) : BE, CH, DE, FR, GB, GR, IT, LI, LU, NL, SE)

1. A compound having the formula (XV): 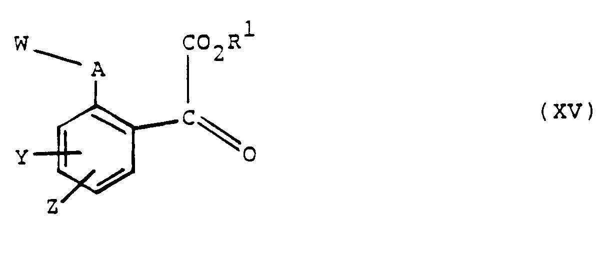 wherein W is a substituted pyridinyl or substituted pyrimidinyl group linked to A by any one of its ring carbon atoms and bearing one or more substituents selected from halogen atoms, C_{1 -6} alkyl itself optionally substituted with halo, phenyl or phenoxy, C₂-₆ alkenyl, phenyl(C₂-₆)alkenyl, C₂-₆ alkynyl, C₁₋₆ alkoxy itself optionally substituted with halo, phenyl or phenoxy, phenoxy, pyridinyloxy, pyrimidinyloxy, phenyl, pyridinyl, pyrimidinyl, nitro, cyano, -NR'R", -NHCOR', -CONR'R", -OCOR', -CO₂R', -COR' or S(O)ₘR' groups, wherein m is 0, 1 or 2 and R' and R" are as defined below; A is either an oxygen atom or S(O)ₙ wherein n is 0, 1 or 2; Y and Z, which are the same or different, are hydrogen or halogen atoms, or C₁ -₆ alkyl, C₁ -₄ alkyl optionally substituted with halo, C₁ -₆ alkoxy or phenyl, C₂-₆ alkenyl, phenyl, C₂-₆ alkynyl, C₁₋₆ alkoxy, C₁₋₄ alkoxy optionally substituted with halo or Ci-₆ alkoxy, phenoxy, phenyl(Cᵢ-₆)alkoxy, -OCOR', -NR'R", -NHCOR', nitro, cyano, -C0₂R³, -CONR4R5, or -COR⁶ groups, wherein R', R" and R³ to R⁶ are as defined below; R¹ is a C₁₋₄ alkyl or C₁₋₄ haloalkyl group; R³, R⁴, R⁵ and R⁶, which are the same or different, are hydrogen atoms or C₁₋₆ alkyl, C₃₋₆ cycloalkyl, C₃₋₆ cycloalkyl(C₁₋₄)alkyl, C₂₋₆ alkenyl, C₂₋₆ alkynyl, phenyl or phenyl(C₁₋₆)-alkyl; R' and R" are independently hydrogen, C₁₋₄ alkyl, C₁₋₄ alkoxy, C₁₋₄ alkylthio, C₃₋₆ cycloalkyl, C₃₋₆ cycloalkyl(C1-4)alkyl, phenyl or benzyl, in which the phenyl and benzyl groups are optionally substituted with halogen, C₁ -₄ alkyl or C₁ -₄ alkoxy; and wherein any of the foregoing phenyl or heteroaryl moieties of Y and Z and of the substituents of W, except where otherwise stated for R' and R", are optionally substituted by one or more of the following: halogen, hydroxy, C₁₋₄ alkyl, C₁₋₄ alkoxy, halo(C₁₋₄)alkyl, halo(C₁₋₄)alkoxy, C₁₋₄ alkylthio, C₁₋₄ alkoxy(C₁₋₄)alkyl, C₃₋₆ cycloalkyl, C₃₋₆ cycloalkyl(C₁₋₄)alkyl, phenyl, phenoxy, phenyl(C₁₋₄)alkyl, phenyl(C₁₋₄)alkoxy, phenoxy(C₁₋₄)alkyl, cyano, thiocyanato, nitro, -NR'R", -NHCOR', -NHCONR'R", -CONR'R", -COOR', -OS0₂R', -S0₂R', -COR', -OCOR', -CR' = NR" or -N = CR'R" wherein R' and R" are as defined above; provided that when W is 5-trifluoromethylpyridin-2-yl, A is oxygen and R¹ is methyl, Y and Z are not both hydrogen, Y is not F, Cl, methyl, nitro, 5-CF₃, or 4-(CH₃)₂N if Z is hydrogen and Y and Z together are not 3-nitro-5-chloro, 3,5-dinitro or 4,5-dimethoxy.

2. A compound as claimed in claim 1 having the formula (XV): 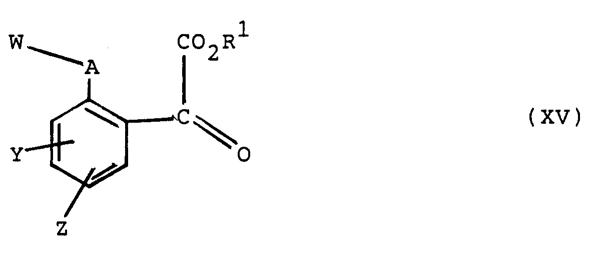 wherein W, A and R¹ are as defined in claim 1; Y and Z, which are the same or different, are hydrogen, fluorine, chlorine or bromine atoms, or C₁ -₄ alkyl (optionally substituted with halogen, C₁ -₄ alkoxy or phenyl), C₂-₅ alkenyl, C₂-₅ alkynyl, phenyl, C₁₋₄ alkoxy (optionally substituted with halogen, C₁₋₄ alkoxy or phenyl), phenoxy, benzyloxy or mono- or di(C₁-₆)alkylamino groups; wherein the phenyl moieties of Y and Z of any of the foregoing are optionally substituted with one or more fluorine, chlorine or bromine atoms, or phenyl, C₁ -₄ alkyl, C₁ -₄ alkoxy, nitro, amino, cyano, hydroxyl or carboxyl groups.

3. A compound according to claim 1 or 2 wherein R¹ is a methyl group.

4. A compound according to any one of the preceding claims wherein Y and Z are both hydrogen atoms.

5. A compound according to any one of the preceding claims wherein A is an oxygen atom.

6. A compound of the formula (XVb): 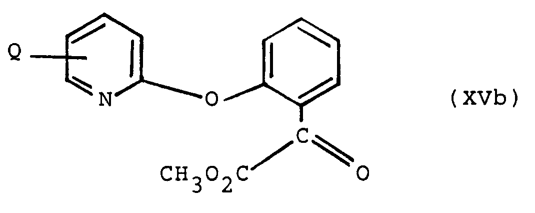 wherein Q is methyl, trifluoromethyl, (but not 5-trifluoromethyl), methoxy, fluorine, chlorine or bromine.

7. A process for preparing a compound of formula (XV) according to claim 1, which comprises treating a compound of formula (XVlla): 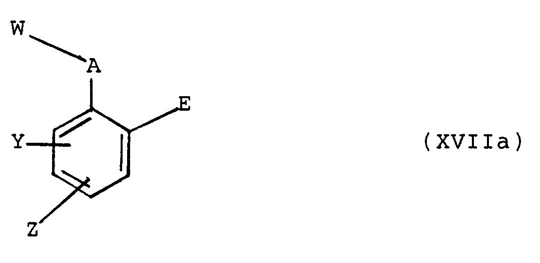 wherein W, A, Y and Z are as defined in claim 1 and E is Mgl, MgBr, MgCI or Li, with an oxalate of formula (XVIII): 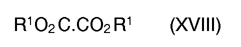 wherein R¹ is as defined in claim 1, in a suitable solvent.

## Claims (Claims for the following Contracting State(s) : AT, ES)

1. A process for preparing a compound having the formula (XV): 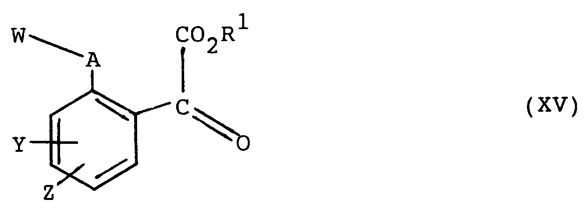 wherein W is a substituted pyridinyl or substituted pyrimidinyl group linked to A by any one of its ring carbon atoms and bearing one or more substituents selected from halogen atoms, C_{1 -6} alkyl itself optionally substituted with halo, phenyl or phenoxy, C₂-₆ alkenyl, phenyl(C₂-₆)alkenyl, C₂-₆ alkynyl, C₁₋₆ alkoxy itself optionally substituted with halo, phenyl or phenoxy, phenoxy, pyridinyloxy, pyrimidinyloxy, phenyl, pyridinyl, pyrimidinyl, nitro, cyano, -NR'R", -NHCOR', -CONR'R", -OCOR', -CO₂R', -COR' or S(O)ₘR' groups, wherein m is 0, 1 or 2 and R' and R" are as defined below; A is either an oxygen atom or S(O)ₙ wherein n is 0, 1 or 2; Y and Z, which are the same or different, are hydrogen or halogen atoms, or C₁ -₆ alkyl, C₁ -₄ alkyl optionally substituted with halo, C₁ -₆ alkoxy or phenyl, C₂-₆ alkenyl, phenyl, C₂-₆ alkynyl, C₁₋₆ alkoxy, C₁₋₄ alkoxy optionally substituted with halo or Ci-₆ alkoxy, phenoxy, phenyl(Cᵢ-₆)alkoxy, -OCOR', -NR'R", -NHCOR', nitro, cyano, -C0₂R³ , -CONR4R5, or -COR⁶ groups, wherein R', R" and R³ to R⁶ are as defined below; R¹ is a C₁₋₄ alkyl or C₁₋₄ haloalkyl group; R³, R⁴, R⁵ and R⁶, which are the same or different, are hydrogen atoms or C₁₋₆ alkyl, C₃₋₆ cycloalkyl, C₃₋₆ cycloalkyl(C₁₋₄)alkyl, C₂₋₆ alkenyl, C₂₋₆ alkynyl, phenyl or phenyl(C₁₋₆)-alkyl; R' and R" are independently hydrogen, C₁₋₄ alkyl, C₁₋₄ alkoxy, C₁₋₄ alkylthio, C₃₋₆ cycloalkyl, C₃₋₆ cycloalkyl(C₁₋₄)alkyl, phenyl or benzyl, in which the phenyl and benzyl groups are optionally substituted with halogen, C₁ -₄ alkyl or C₁ -₄ alkoxy; and wherein any of the foregoing phenyl or heteroaryl moieties of Y and Z and of the substituents of W, except where otherwise stated for R' and R", are optionally substituted by one or more of the following: halogen, hydroxy, C₁₋₄ alkyl, C₁₋₄ alkoxy, halo(C₁₋₄)alkyl, halo(C₁₋₄)alkoxy, C₁₋₄ alkylthio, C₁₋₄ alkoxy(C₁₋₄)alkyl, C₃₋₆ cycloalkyl, C₃₋₆ cycloalkyl(C₁₋₄)alkyl, phenyl, phenoxy, phenyl(C₁₋₄)alkyl, phenyl(C₁₋₄)alkoxy,phenoxy(C₁₋₄)alkyl, cyano, thiocyanato, nitro, -NR'R", -NHCOR', -NHCONR'R", -CONR'R", -COOR', -OS0₂ R', -S0₂ R', -COR', -OCOR', -CR'=NR'' or -N = CR'R" wherein R' and R" are as defined above;

provided that when W is 5-trifluoromethylpyridin-2-yl, A is oxygen and R¹ is methyl, Y and Z are not both hydrogen, Y is not F, Cl, methyl, nitro, 5-CF₃, or 4-(CH₃)₂N if Z is hydrogen and Y and Z together are not 3-nitro-5-chloro, 3,5-dinitro or 4,5-dimethoxy;

which process comprises treating a compound of formula (XVlla): 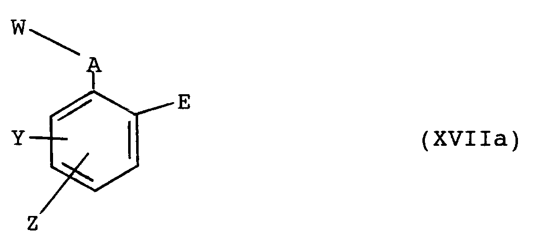 wherein W, A, Y and Z are as defined above, and E is Mgl, MgBr, MgCI or Li, with an oxalate of formula (XVIII): 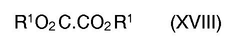 wherein R¹ is as defined above, in a suitable solvent.

2. A process as claimed in claim 1 wherein in the compound of formula (XV) W, A and R¹ are as defined in claim 1; and Y and Z, which are the same or different, are hydrogen, fluorine, chlorine or bromine atoms, or C₁₋₄ alkyl (optionally substituted with halogen, C₁₋₄ alkoxy or phenyl), C₂-₅ alkenyl, C₂-₅ alkynyl, phenyl, C₁ -₄ alkoxy (optionally substituted with halogen, C₁ -₄ alkoxy or phenyl), phenoxy, benzyloxy or mono- or di(Cᵢ -₆)alkylamino groups; wherein the phenyl moieties of Y and Z of any of the foregoing are optionally substituted with one or more fluorine, chlorine or bromine atoms, or phenyl, C₁ -₄ alkyl, C₁ -₄ alkoxy, nitro, amino, cyano, hydroxyl or carboxyl groups.

3. A process according to claim 1 or 2 wherein in the compound of formula (XV) R¹ is a methyl group.

4. A process according to any one of the preceding claims wherein in the compound of formula (XV) Y and Z are both hydrogen atoms.

5. A process according to any one of the preceding claims wherein in the compound of formula (XV) A is an oxygen atom.

6. A process according to claim 1 wherein the compound of formula (XV) is a compound of the formula (XVb): 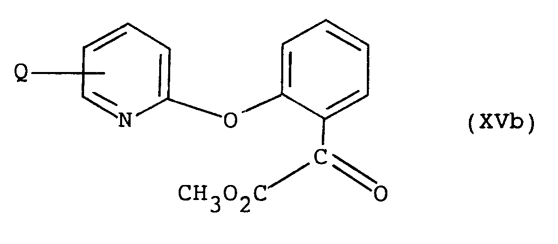 wherein Q is methyl, trifluoromethyl, (but not 5-trifluoromethyl), methoxy, fluorine, chlorine or bromine.

## Patentansprüche (Patentansprüche für folgende(n) Vertragsstaat(en) : BE, CH, DE, FR, GB, GR, IT, LI, LU, NL, SE)

1. Verbindungen der Formel (XV) 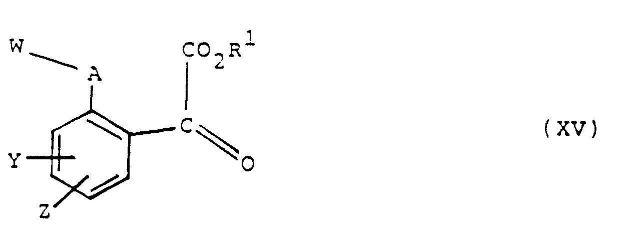 worin W für eine substituierte Pyridinyl- oder Pyrimidinyl-Gruppe steht, die an A über eines ihrer Ringkohlenstoffatome gebunden ist und die einen oder mehrere Substituenten trägt, die ausgewählt sind aus Halogenatomen, C₁₋₆-Alkyl, das selbst gegebenenfalls mit Halogeno, Phenyl oder Phenoxy substituiert ist, C₂-₆-Alkenyl, Phenyl-C₂-₆-alkenyl, C₂-₆-Alkinyl, Cᵢ-₆-Alkoxy, das gegebenenfalls selbst mit Halogeno, Phenyl oder Phenoxy substituiert ist, Phenoxy, Pyridinyloxy, Pyrimidinyloxy, Phenyl, Pyridinyl, Pyrimidinyl, Nitro, Cyano, -NR'R", -NHCOR', -CONR'R", -OCOR', -C0₂R', -COR' oder -S(O)ₘR', worin m 0, 1 oder 2 bedeutet und R' und R" die weiter unten angegebenen Bedeutungen besitzen; A für ein Sauerstoffatom oder S(O)ₙ steht, worin n 0, 1 oder 2 bedeutet; Y und Z, welche gleich oder verschieden sein können, für Wasserstoff- oder Halogenatome oder C₁₋₆-Alkyl C1-4-Alkyl, das gegebenenfalls mit Halogeno, C₁₋₆-Alkoxy oder Phenyl substituiert ist, C₂-₆-Alkenyl, Phenyl C₂-₆-Alkinyl, C₁₋₆-Alkoxy, C₁₋₄-Alkoxy, das gegebenenfalls mit Halogeno oder C₁₋₆-Alkoxy substituiert ist, Phenoxy, Phenyl-Ci-₆-alkoxy, -OCOR', -NR'R", -NHCOR', Nitro, Cyano, -C0₂R³, -CONR⁴R⁵ oder -COR⁶ stehen, worin R', R" und R³ bis R⁶ die weiter unten angegebenen Bedeutungen besitzen; R¹ für C₁₋₄-Alkyl oder C₁₋₄-Halogenoalkyl steht; R³ R⁴, R⁵ und R⁶, welche gleich oder verschieden sein können, für Wasserstoffatome oder Ci-6-Alkyl, C₃-₆-Cycloalkyl, C₃-₆-Cycloalkyl-C₁₋₄-alkyl, C₂-₆-Alkenyl, C₂-₆-Alkinyl, Phenyl oder Phenyl-C₁-₆-alkyl stehen; und R' und R" jeweils für Wasserstoff, C₁₋₄-Alkyl, C₁₋₄-Alkoxy, C₁₋₄-Alkylthio, C₃-₆-Cycloalkyl, C₃₋₆-Cycloalkyl-C₁₋₄-alkyl, Phenyl oder Benzyl stehen, wobei die Phenyl- und Benzyl-Gruppen gegebenenfalls mit Halogen, C₁₋₄-Alkyl oder C₁₋₄-Alkoxy substituiert sind;
und wobei die oben angegebenen Phenyl- oder Heteroaryl-Teile von Y und Z und der Substituenten von W, außer wenn für R' und R" etwas anderes angegeben ist, gegebenenfalls einen oder mehrere der folgenden Substituenten tragen:
Halogen, Hydroxy, C₁₋₄-Alkyl, C₁ -₄-Alkoxy, Halogeno-Cᵢ -₄-alkyl, Halogeno-C₁₋₄-alkoxy, C₁ -₄-Alkylthio, C₁₋₄-Alkoxy-C₁₋₄-alkyl, C₃₋₆-Cycloalkyl, C₃₋₆-Cycloalkyl-C₁₋₄-alkyl, Phenyl, Phenoxy, Phenyl-C₁₋₄-alkyl, Phenyl-C1-₄-alkoxy, Phenoxy-C₁₋₄-alkyl, Cyano, Thiocyanoto, Nitro, -NR'R", -NHCOR', -NHCONR'R", -CONR'R", -COOR', -OS0₂R', -S0₂R', -COR', -OCOR', -CR'=NR" oder -N=CR'R", worin R' und R" die oben angegebenen Bedeutungen besitzen;
mit der Maßgabe, daß, wenn W für 5-Trifluoromethylpyridin-2-yl steht, A für Sauerstoff steht und R¹ für Methyl steht, Y und Z nicht beide Wasserstoff bedeuten, Y nicht F, Cl, Methyl, Nitro, 5-CF₃ oder 4-(CH₃)₂N, sofern Z Wasserstoff ist, bedeutet und Y und Z nicht gemeinsam 3-Nitro-5-chloro, 3,5-Dinitro oder 4,5-Dimethoxy bedeuten.

2. Verbindungen nach Anspruch 1 der Formel (XV) 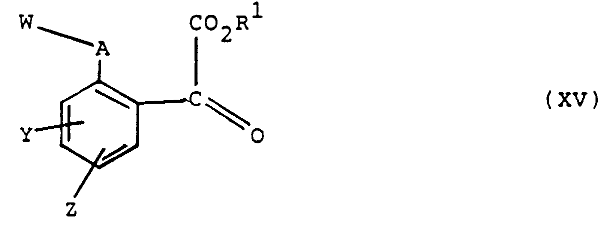 worin W, A und R¹ die in Anspruch 1 angegebenen Bedeutungen besitzen; und Y und Z, welche gleich oder verschieden sein können, für Wasserstoff-, Fluor-, Chlor- oder Bromatome oder C₁₋₄-Alkyl (gegebenenfalls mit Halogen, C₁₋₄-Alkoxy oder Phenyl substituiert), C₂-₅-Alkenyl, C₂-₅-Alkinyl, Phenyl, C₁₋₄-Alkoxy (gegebenenfalls mit Halogen, C₁₋₄-Alkoxy oder Phenyl substituiert), Phenoxy, Benzyloxy oder Mono- oder Di-C₁₋₆-alkylamino stehen; wobei die Phenyl-Teile von Y und Z gegebenenfalls einen oder mehrere der folgenden Substituenten tragen: Fluor-, Chlor- oder Bromatome oder Phenyl, C₁₋₄-Alkyl, C₁ -₄-Alkoxy, Nitro, Amino, Cyano, Hydroxy oder Carboxy.

3. Verbindungen nach Anspruch 1 oder 2, worin R¹ für Methyl steht.

4. Verbindungen nach einem der vorhergehenden Ansprüche, worin Y und Z beide für Wasserstoffatome stehen.

5. Verbindungen nach einem der vorhergehenden Ansprüche, worin A für ein Sauerstoffatom steht.

6. Verbindungen der Formel (XVb) 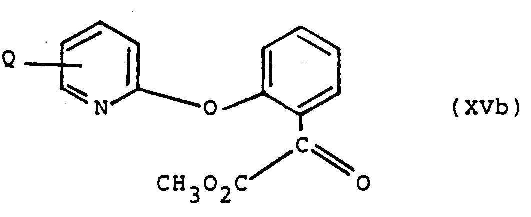 worin Q für Methyl Trifluoromethyl (aber nicht 5-Trifluoromethyl), Methoxy, Fluor, Chlor oder Brom steht.

7. Verfahren zur Herstellung einer Verbindung der Formel (XV) von Anspruch 1, bei welchem eine Verbindung der Formel (XVIla), 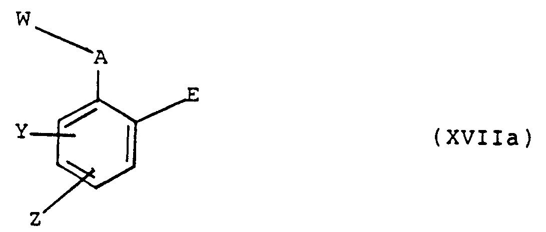 worin W, A, Y und Z die in Anspruch 1 angegebenen Bedeutungen besitzen und E für MgJ, MgBr, MgCI oder Li steht, mit einem Oxalat der Formel (XVIII)
R¹O₂C.CO₂R¹ (XVIII)
worin R¹ die in Anspruch 1 angegebene Bedeutung besitzt, in einem geeigneten Lösungsmittel umgesetzt wird.

## Patentansprüche (Patentansprüche für folgende(n) Vertragsstaat(en) : AT, ES)

1. Verfahren zur Herstellung einer Verbindung der Formel (XV) 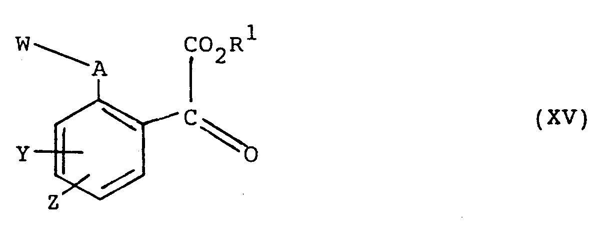 worin W für eine substituierte Pyridinyl- oder Pyrimidinyl-Gruppe steht, die an A über eines ihrer Ringkohlenstoffatome gebunden ist und die einen oder mehrere Substituenten trägt, die ausgewählt sind aus Halogenatomen, C₁₋₆Alkyl, das selbst gegebenenfalls mit Halogeno, Phenyl oder Phenoxy substituiert ist, C₂-₆-Alkenyl, Phenyl-C₂-₆-alkenyl, C₂-₆-Alkinyl, Cᵢ-₆-Alkoxy, das gegebenenfalls selbst mit Halogeno, Phenyl oder Phenoxy substituiert ist, Phenoxy, Pyridinyloxy, Pyrimidinyloxy, Phenyl, Pridinyl, Pyrimidinyl, Nitro, Cyano, -NR'R", -NHCOR', -CONR'R", -OCOR', -C0₂R', -COR' oder -S(O)ₘR', worin m 0, 1 oder 2 bedeutet und R' und R" die weiter unten gegegebenen Bedeutungen besitzen; A für ein Sauerstoffatom oder S(O)ₙ steht, worin n 0, 1 oder 2 bedeutet; Y und Z, welche gleich oder verschieden sein können, für Wasserstoff- oder Halogenatome oder C₁₋₆-Alkyl, C₁₋₄-Alkyl, das gegebenenfalls mit Halogeno, C₁₋₆-Alkoxy oder Phenyl substituiert ist, C₂-₆-Alkenyl, Phenyl, C₂-₆-Alkinyl, C₁-₆-Alkoxy, C₁₋₄-Alkoxy, das gegebenenfalls mit Halogeno oder C₁-₆-Alkoxy substituiert ist, Phenoxy, Phenyl-C₁-₆-alkoxy, -OCOR', -NR'R", -NHCOR', Nitro, Cyano,-C0₂R³, -CONR⁴R⁵ oder -COR⁶ stehen, worin R', R" und R³ bis R⁶ die weiter unten angegebenen Bedeutungen besitzen; R¹ für C₁₋₄-Alkyl oder C₁₋₄-Halogenoalkyl steht; R³, R⁴, R⁵ und R⁶, welche gleich oder verschieden sein können, für Wasserstoffatome oder C₁₋₆-Alkyl, C₃-₆-Cycloalkyl, C₃₋₆-Cycloalkyl-C₁₋₄-alkyl, C₂-₆-Alkenyl, C₂-₆-Alkinyl, Phenyl oder Phenyl-C₁₋₆-alkyl stehen; und R' und R" jeweils für Wasserstoff, C₁₋₄-Alkyl C₁₋₄-Alkoxy, C₁₋₄-Alkylthio, C3-6-Cycloalkyl, C₃₋₆-Cycloalkyl-C₁₋₄-alkyl, Phenyl oder Benzyl stehen, wobei die Phenyl- und Benzyl-Gruppen gegebenenfalls mit Halogen, C₁₋₄-Alkyl oder C₁₋₄-Alkoxy substituiert sind;
und wobei die oben angegebenen Phenyl- oder Heteroaryl-Teile von Y und Z und der Substituenten von W, außer wenn für R' und R" etwas anderes angegeben ist, gegebenenfalls einen oder mehrere der folgenden Substituenten tragen:
Halogen, Hydroxy, C₁₋₄-Alkyl, C₁ -₄-Alkoxy, Halogeno-Cᵢ -₄-alkyl, Halogeno-C₁ -₄-alkoxy, C₁ -₄-Alkylthio, C₁₋₄-Alkoxy-C₁₋₄-alkyl, C₃₋₆-Cycloalkyl, C₃₋₆-Cycloalkyl-C₁₋₄-alkyl, Phenyl, Phenoxy, Phenyl-C₁₋₄-alkyl, Phenyl-C1-₄-alkoxy, Phenoxy-C₁₋₄-alkyl, Cyano, Thiocyanato, Nitro, -NR'R", -NHCOR', -NHCONR'R", -CONR'R", -COOR', -OS0₂R', -S0₂R', -COR', -OCOR', -CR'=NR" oder -N=CR'R", worin R' und R" die oben angegebenen Bedeutungen besitzen;
mit der Maßgabe, daß, wenn W für 5-Trifluoromethylpyridin-2-yl steht, A für Sauerstoff steht und R¹ für Methyl steht, Y und Z nicht beide Wasserstoff bedeuten, Y nicht F, Cl, Methyl, Nitro, 5-CF₃ oder 4-(CH₃)₂N, sofern Z Wasserstoff ist, bedeutet und Y und Z nicht gemeinsam 3-Nitro-5-chloro, 3,5-Dinitro oder 4,5-Dimethoxy bedeuten; bei welchem Verfahren eine Verbindung der Formel (XVIIa) 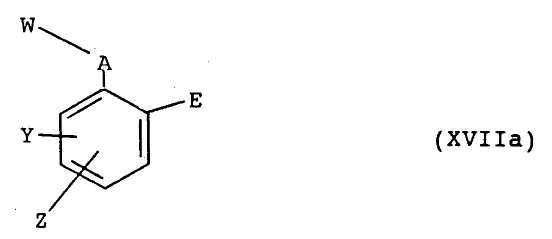 worin W, A, Y und Z die oben angegebenen Bedeutungen besitzen und E für MgJ, MgBr, MgCI oder Li steht, mit einem Oxalat der Formel (XVIII) 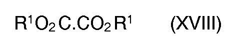 worin R¹ die oben angegebene Bedeutung besitzt, in einem geeigneten Lösungsmittel umgesetzt wird.

2. Verfahren nach Anspruch 1, bei welchem in der Verbindung der Formel (XV) W, A und R¹ die in Anspruch 1 angegebenen Bedeutungen besitzen; und Y und Z, welche gleich oder verschieden sein können, für Wasserstoff-, Fluor-, Chlor- oder Bromatome oder C₁₋₄-Alkyl (gegebenenfalls mit Halogen, C₁₋₄-Alkoxy oder Phenyl substituiert), C₂-₅-Alkenyl C₂-₅-Alkinyl, Phenyl, C₁₋₄-Alkoxy (gegebenenfalls mit Halogen, C₁₋₄-Alkoxy oder Phenyl substituiert), Phenoxy, Benzyloxy oder Mono- oder Di-C₁-₆- alkylamino stehen; wobei die Phenyl-Teile von Y und Z gegebenenfalls einen oder mehrere der folgenden Substituenten tragen: Fluor-, Chlor- oder Bromatome oder Phenyl C₁₋₄-Alkyl, C₁₋₄-Alkoxy, Nitro, Amino, Cyano, Hydroxy oder Carboxy.

3. Verfahren nach Anspruch 1 oder 2, bei welchem in der Verbindung der Formel (XV) R¹ für Methyl steht.

4. Verfahren nach einem der vorhergehenden Ansprüche, bei welchem in der Verbindung der Formel (XV) Y und Z beide für Wasserstoffatome stehen.

5. Verfahren nach einem der vorhergehenden Ansprüche, bei welchem in der Verbindung der Formel (XV) A für ein Sauerstoffatom steht.

6. Verfahren nach Anspruch 1 bei welchem die Verbindung der Formel (XV) eine Verbindung der Formel (XVb) ist, 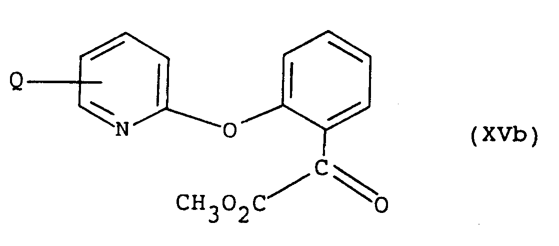 worin Q für Methyl, Trifluoromethyl (aber nicht 5-Trifluoromethyl), Methoxy, Fluor, Chlor oder Brom steht.

## Revendications (Revendications pour l'(les) Etat(s) contractant(s) suivant(s) : suivants : BE, CH, DE, FR, GB, GR, IT, LI, LU, NL, SE)

1. Composé de formule (XV) : 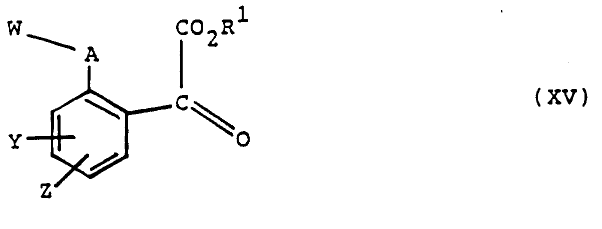 dans laquelle W représente un groupe pyridinyle substitué ou pyrimidinyle substitué lié à A par l'un quelconque de ses atomes de carbone du noyau et portant un ou plusieurs substituants choisis entre des atomes d'halogènes, un groupe alkyle en Ci à C₆ lui-même facultativement substitué avec un groupe halogéno, phényle ou phénoxy, alcényle en C₂ à C₆, phényl-(alcényle en C₂ à C₆), alcynyle en c₂ à C₆, alkoxy en Ci à C₆ lui-même facultativement substitué avec des groupes halogéno, phényle ou phénoxy, phénoxy, pyridinyloxy, pyrimidinyloxy, phényle, pyridinyle, pyrimidinyle, nitro, cyano, -NR'R", -NHCOR', -CONR'R", -OCOR', -C0₂R', -COR' ou S(O)ₘR', dans lesquels m est égal à 0, 1 ou 2 et R' et R" répondent aux définitions mentionnées ci-dessous ; A représente un atome d'oxygène ou un groupe S(O)ₙ dans lequel n est égal à 0, 1 ou 2 ; Y et Z, qui sont identiques ou différents, représentent des atomes d'hydrogène ou d'halogènes, ou des groupes alkyle en Ci à C₆, alkyle en Ci à C₄ facultativement substitués avec un halogène, alkoxy en Ci à C₆ ou phényle, alcényle en C₂ à C₆, phényle, alcynyle en C₂ à C₆, alkoxy en Ci à C₆, alkoxy en Ci à C₄ facultativement substitué avec un halogène ou alkoxy en Ci à C₆, phénoxy, phényl-(alkoxy en Ci à C₆), -OCOR', -NR'R", -NHCOR', nitro, cyano, -C0₂R³, -CONR⁴R⁵ ou -COR⁶, dans lesquels R', R" et R³ à R⁶ répondent aux définitions mentionnées ci-dessous ; R¹ représente un groupe alkyle en Ci à C₄ ou halogénalkyle en Ci à C₄ ; R³, R⁴, R⁵ et R⁶, qui sont identiques ou différents, représentent des atomes d'hydrogène ou des groupes alkyle en Ci à C₆, cycloalkyle en C₃ à C₆, (cycloalkyle en C₃ à C₆)-(alkyle en C₁ à C₄), alcényle en C₂ à C₆, alcynyle en C₂ à C₆, phényle ou phényl-(alkyle en C₁ à C₆) ; R' et R" représentent, indépendamment, l'hydrogène, des groupes alkyle en C₁ à C₄, alkoxy en C₁ à C₄, alkylthio en C₁ à C₄, cycloalkyle en C₃ à C₆, (cycloalkyle en C₃ à C₆)-(alkyle en C₁ à C₄), phényle ou benzyle, les groupes phényle et benzyle étant facultativement substitués avec des groupes halogéno, alkyle en C₁ à C₄ ou alkoxy en C₁ à C₄ ; et dans lequel n'importe lesquels des groupements phényle ou hétéro-aryle précités de Y et Z et des substituants de W, sauf spécification contraire pour R' et R", sont facultativement substitués avec un ou plusieurs des groupes suivants : halogéno, hydroxy, alkyle en C₁ à C₄, alkoxy en Ci à C₄, halogénalkyle en Ci à C₄, halogénalkoxy en Ci à C₄, alkylthio en Ci à C₄, (alkoxy en Ci à C₄)-(alkyle en Ci à C₄), cycloalkyle en C₃ à C₆, (cycloalkyle en C₃ à C₆)-(alkyle en Ci à C₄), phényle, phénoxy, phényl-(alkyle en Ci à C₄), phényl-(alkoxy en Ci à C₄), phénoxy-(alkyle en C₁ à C₄), cyano, thiocyanato, nitro, -NR'R", -NHCOR', -NHCONR'R", -CONR'R", -COOR', -OS0₂R', -S0₂ R', -COR', -OCOR', -CR'=NR'' ou -N = CR'R", dans lesquels R' et R" répondent aux définitions précitées ; sous réserve que, lorsque W représente un groupe 5-trifluorométhylpyridine-2-yle, A représente l'oxygène et R¹ représente un groupe méthyle, Y et Z ne représentent pas l'un et l'autre l'hydrogène, Y ne représente pas F, CI, un groupe méthyle, nitro, 5-CF₃ ou 4-(CH₃)₂N si Z représente l'hydrogène, et Y et Z ne représentent pas conjointement un groupe 3-nitro-5-chloro, 3,5-dinitro ou 4,5-diméthoxy.

2. Composé suivant la revendication 1, répondant à la formule (XY) : 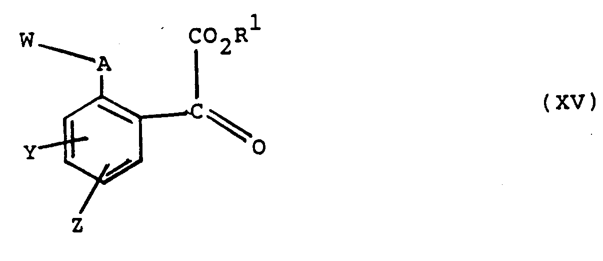 dans laquelle W, A et R¹ répondent aux définitions figurant dans la revendication 1 ; Y et Z, qui sont identiques ou différents, représentent l'hydrogène ou des atomes de fluor, de chlore ou de brome, ou des groupes alkyle en C₁ à C₄ (facultativement substitués avec des groupes halogéno, alkoxy en C₁ à C₄ ou phényle), alcényle en C₂ à Cs, alcynyle en C₂ à Cs, phényle, alkoxy en C₁ à C₄ (facultativement substitués avec des groupes halogéno, alkoxy en C₁ à C₄ ou phényle), phénoxy, benzyloxy ou mono-ou di-(alkyle en C₁ à C₆)amino ; les groupes phényle de Y et Z étant facultativement substitués avec un ou plusieurs atomes de fluor, de chlore ou de brome ou groupes phényle, alkyle en C₁ à C₄, alkoxy en C₁ à C₄, nitro, amino, cyano, hydroxyle ou carboxyle.

3. Composé suivant la revendication 1 ou 2, dans lequel R¹ représente un groupe méthyle.

4. Composé suivant l'une quelconque des revendications précédentes, dans lequel Y et Z représentent l'un et l'autre des atomes d'hydrogène.

5. Composé suivant l'une quelconque des revendications précédentes, dans lequel A représente un atome d'oxygène.

6. Composé de formule (XVb) : 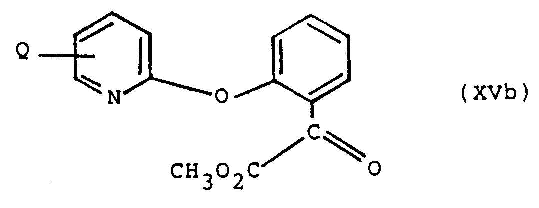 dans laquelle Q représente un groupe méthyle, trifluorométhyle, (mais non 5-trifluorométhyle), méthoxy, fluoro, chloro ou bromo.

7. Procédé de préparation d'un composé de formule (XV) suivant la revendication 1, qui comprend le traitement d'un composé de formule (XVlla) : 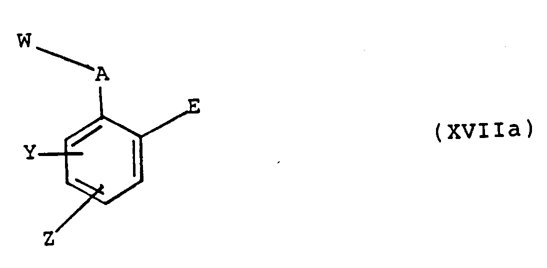 dans laquelle W, A, Y et Z répondent aux définitions mentionnées dans la revendication 1 et E représente Mgl, MgBr, MgCl ou Li, avec un oxalate de formule (XVIII) :
R¹O₂C.CO₂R¹ (XVIII)
dans laquelle R¹ répond à la définition mentionnée dans la revendication 1, dans un solvant convenable.

## Revendications (Revendications pour l'(les) Etat(s) contractant(s) suivant(s) : suivants ; AT, ES)

1. Procédé de préparation d'un composé répondant à la formule (XV) : 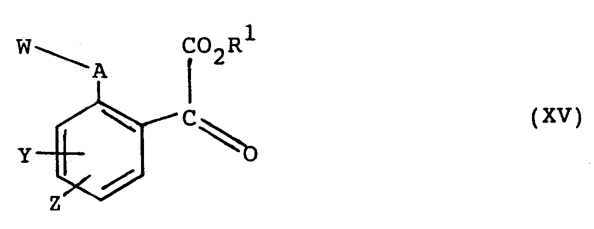 dans laquelle W représente un groupe pyridinyle substitué ou pyrimidinyle substitué lié à A par l'un quelconque de ses atomes de carbone du noyau et portant un ou plusieurs substituants choisis entre des atomes d'halogènes, un groupe alkyle en Ci à C₆ lui-même facultativement substitué avec des groupes halogéno, phényle ou phénoxy, alcényle en C₂ à C₆, phényl-(alcényle en C₂ à C₆), alcynyle en C₂ à C₆, alkoxy en Ci à C₆ lui-même facultativement substitué avec des groupes halogéno, phényle ou phénoxy, phénoxy, pyridinyloxy, pyrimidinyloxy, phényle, pyridinyle, pyrimidinyle, nitro, cyano, -NR'R", -NHCOR', -CONR'R", -OCOR', -C0₂R', -COR' ou S(O)ₘR', dans lesquels m est égal à 0, 1 ou 2 et R' et R" répondent aux définitions mentionnées ci-dessous ; A représente un atome d'oxygène ou un groupe S(O)ₙ dans lequel n est égal à 0, 1 ou 2 ; Y et Z, qui sont identiques ou différents, représentent des atomes d'hydrogène ou d'halogènes, ou des groupes alkyle en Ci à C₆, alkyle en Ci à C₄ facultativement substitué avec des groupes halogéno, alkoxy en Ci à C₆ ou phényle, alcényle en C₂ à C₆, phényle, alcynyle en C₂ à C₆, alkoxy en Ci à C₆, alkoxy en Ci à C₄ facultativement substitué avec des groupes halogéno ou alkoxy en Ci à C₆, phénoxy, phényl-(alkoxy en Ci à C₆), -OCOR', -NR'R", -NHCOR', nitro, cyano, -C0₂R³, -CONR⁴R⁵ ou -COR⁶, dans lesquels R', R" et R³ à R⁶ répondent aux définitions mentionnées ci-dessous ; R¹ représente un groupe alkyle en Ci à C₄ ou halogénalkyle en C₁ à C₄ ; R³, R⁴, R⁵ et R⁶, qui sont identiques ou différents, représentent des atomes d'hydrogène ou des groupes alkyle en Ci à C₆, cycloalkyle en C₃ à C₆, (cycloalkyle en C₃ à C₆)-(alkyle en Ci à C₄), alcényle en C₂ à C₆, alcynyle en C₂ à C₆, phényle ou phényl-(alkyle en Ci à C₆) ; R'et R" représentent, indépendamment, l'hydrogène, des groupes alkyle en Ci à C₄, alkoxy en C₁ à C₄, alkylthio en C₁ à C₄, cycloalkyle en C₃ à C₆, (cycloalkyle en C₃ à C₆)-(alkyle en C₁ à C₄), phényle ou benzyle, les groupes phényle et benzyle étant facultativement substitués avec des groupes halogéno, alkyle en C₁ à C₄ ou alkoxy en C₁ à C₄ ;
et dans laquelle n'importe lesquels des groupes phényle ou hétéro-aryle précités de Y et Z et des substituants de W, sauf spécification contraire pour R' et R", sont facultativement substitués avec un ou plusieurs des groupes suivants : halogéno, hydroxy, alkyle en C₁ à C₄, alkoxy en C₁ à C₄, halogénalkyle en Ci à C₄, halogénalkoxy en Ci à C₄, alkylthio en Ci à C₄, (alkoxy en Ci à C₄)-(alkyle en Ci à C₄), cycloalkyle en C₃ à C₆, (cycloalkyle en C₃ à C₆)-(alkyle en Ci à C₄), phényle, phénoxy, phényl-(alkyle en Ci à C₄), phényl-(alkoxy en Ci à C₄), phénoxy-(alkyle en Ci à C₄), cyano, thiocyanato, nitro, -NR'R", -NHCOR', -NHCONR'R", -CONR'R", -COOR', -OS0₂R', -S0₂R', -COR', -OCOR', -CR' = NR" ou -N = CR'R", dans lesquels R' et R" répondent aux définitions précitées ;
sous réserve que, lorsque W représente un groupe 5-trifluorométhylpyridine-2-yle, A représente l'oxygène et R¹ représente un groupe méthyle, Y et Z ne représentent pas l'un et l'autre l'hydrogène, Y ne représente pas F, CI, un groupe méthyle, nitro, 5-CF₃ ou 4-(CH₃)₂N si Z représente l'hydrogène, et Y et Z ne représentent pas conjointement un groupe 3-nitro-5-chloro, 3,5-dinitro ou 4,5-diméthoxy ;
procédé qui comprend le traitement d'un composé de formule (XVlla) : 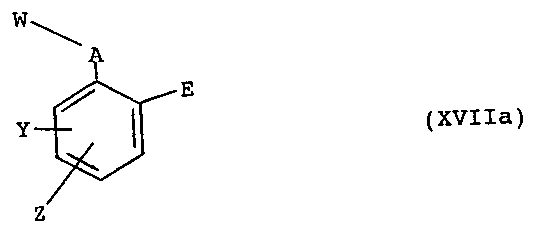 dans laquelle W, A, Y et Z répondent aux définitions précitées, et E représente Mgl, MgBr, MgCl ou Li, avec un oxalate de formule (XVIII) : 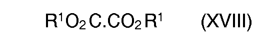
dans laquelle R¹ répond à la définition précitée, dans un solvant convenable.

2. Procédé suivant la revendication 1, dans lequel, dans le composé de formule (XV), W, A et R¹ répondent aux définitions mentionnées dans la revendication 1 ; et Y et Z, qui sont identiques ou différents, représentent des atomes d'hydrogène, de fluor, de chlore ou de brome, ou des groupes alkyle en Ci à C₄ (facultativement substitués avec des groupes halogéno, alkoxy en C₁* à C₄ ou phényle), alcényle en C₂ à Cₛ, alcynyle en C₂ à Cₛ, phényle, alkoxy en C, à C₄ (facultativement substitués avec des groupes halogéno, alkoxy en C₁ à C₄ ou phényle), phénoxy, benzyloxy ou mono-ou di-(alkyle en C₁ à C₆)amino ; les groupes phényle de Y et Z étant facultativement substitués avec un ou plusieurs atomes de fluor, de chlore ou de brome ou groupes phényle, alkyle en C₁ à C₄, alkoxy en C₁ à C₄, nitro, amino, cyano, hydroxyle ou carboxyle.

3. Procédé suivant la revendication 1 ou 2, dans lequel, dans le composé de formule (XV), R¹ représente un groupe méthyle.

4. Procédé suivant l'une quelconque des revendications précédentes, dans lequel, dans le composé de formule (XV), Y et Z représentent l'un et l'autre des atomes d'hydrogène.

5. Procédé suivant l'une quelconque des revendications précédentes, dans lequel, dans le composé de formule (XV), A représente un atome d'oxygène.

6. Procédé suivant la revendication 1, dans lequel le composé de formule (XV) est un composé de formule (XVb) : 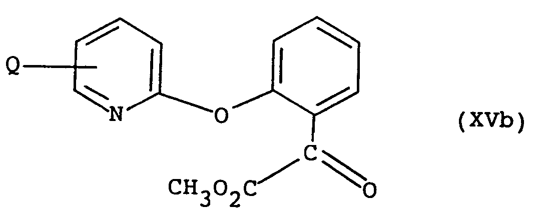
dans laquelle Q représente un groupe méthyle, trifluorométhyle (mais non 5-trifluorométhyle), méthoxy, fluoro, chloro ou bromo.
